(19)

**Europäisches Patentamt**
**European Patent Office**
**Office européen des brevets**

(11)  **EP 3 904 986 B1**

(12)  **EUROPEAN PATENT SPECIFICATION**

(45) Date of publication and mention
of the grant of the patent:
**02.08.2023  Bulletin 2023/31**

(21) Application number: **20382341.4**

(22) Date of filing: **27.04.2020**

(51) International Patent Classification (IPC):
**G05B 23/02** *(2006.01)*     **G01N 33/28** *(2006.01)*

(52) Cooperative Patent Classification (CPC):
**G05B 23/0243;** G01N 33/28

(54) **METHOD, SYSTEM AND COMPUTER PROGRAM PRODUCT FOR THE ONLINE MONITORING OF AN OIL REFINERY**

VERFAHREN, SYSTEM UND COMPUTERPROGRAMMPRODUKT ZUR ONLINE-ÜBERWACHUNG EINER ÖLRAFFINERIE

PROCÉDÉ, SYSTÈME ET PRODUIT PROGRAMME INFORMATIQUE POUR LA SURVEILLANCE EN LIGNE D'UNE RAFFINERIE DE PÉTROLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.11.2021  Bulletin 2021/44**

(73) Proprietor: **Petroleos del Norte, S.A.**
**48550 Muskiz (Vizcaya) (ES)**

(72) Inventors:
• **Cerezo Rancaño, Juan Francisco**
  **48550 Muskiz (Vizcaya) (ES)**
• **Salas Sierra, Beatriz**
  **48550 Muskiz (Vizcaya) (ES)**
• **Regil Herrero, Ainhoa**
  **48550 Muskiz (Vizcaya) (ES)**
• **Unzueta Etxeita, Elías**
  **48550 Muskiz (Vizcaya) (ES)**
• **Orbe Guijarro, Lucía**
  **48550 Muskiz (Vizcaya) (ES)**
• **Maiza, Mikel**
  **20009 San Sebastian (ES)**

• **Odriozola, Juan**
  **20009 San Sebastian (ES)**
• **Artetxe, Arkaitz**
  **20009 San Sebastian (ES)**
• **García, Igor**
  **20009 San Sebastian (ES)**
• **Flórez, Julián**
  **20009 San Sebastian (ES)**
• **Quartulli, Marco**
  **20009 San Sebastian (ES)**
• **Goicolea, Juan**
  **28220 Majadahonda, Madrid (ES)**
• **Villanueva, Unai**
  **20009 San Sebastian (ES)**

(74) Representative: **Balder IP Law, S.L.**
**Paseo de la Castellana 93**
**5ª planta**
**28046 Madrid (ES)**

(56) References cited:
**US-A1- 2014 180 650     US-A1- 2018 348 189**
**US-A1- 2020 033 317**

**Description**

## TECHNICAL FIELD

The present invention relates to the field of oil processing. In particular, it relates to processes for refining crude oil. The invention envisages a method and system for the online monitoring of an oil refinery plant by means of a soft sensor, such as monitoring a production operation parameter in oil refining processes.

## STATE OF THE ART

In the field of oil processing, the optimum performance management of an oil refinery is a continuous target. Ultimately, economic performance is a key metrics which depends on the complexity of the refinery plant and on the economic impact of every process of the plant. In an oil refinery plant, the coker unit is a key process in terms of economic importance and therefore its optimal operation is a must. Optimal control of the operation of the coker unit highly depends on a critical production operation parameter: the penetration value of the residue of the vacuum distillation unit. The vacuum distillation unit is the unit in the oil refinery plant in which secondary refining processes following the main one -performed at the main distillation units-, occur. Its residue, a crude oil blend stream, is inputted to the coker unit, which is optimally operated if an optimum penetration value of the residue of the vacuum distillation unit is obtained.

The penetration value of a crude oil blend is, in general terms, a measure of its hardness or consistency. More specifically, the penetration value of a crude oil blend is defined as the vertical distance traversed or penetrated by a standard needle into the crude oil blend under specific conditions of load, time and temperature. This distance is measured in tenths of a millimetre (mm) (1mm = $10^{-3}$ meters (m)). The shorter the distance, the lower is the penetration value so, the harder is the crude oil blend; the longer the distance, the higher is the penetration value so, the softer is the crude oil blend. Thus, the coker unit is optimally operated if a residue of the vacuum distillation unit having optimum hardness or consistency is obtained. Therefore, getting an optimum penetration value implies optimally operating the vacuum distillation unit. In order to get an optimum penetration value, the following must be done: (i) firstly, a sensitive, correct setup of initial values of a plurality of production operation parameters must be estimated in production planning time; (ii) secondly, robust and accurate control loops must be configured; (iii) thirdly, a frequent and reliable feedback of the penetration value is needed.

Currently, the standard method for measuring the penetration value is by means of a tedious, costly and time-consuming laboratory analysis: the so-called needle penetration method. It requires specific laboratory equipment including a container, a needle, a water bath, a transfer dish, a penetration apparatus, a weight, a temperature measuring device and a time measuring device. The method includes (i) preparing the crude oil blend sample by heating it to a pouring consistency and stirring it until it gets homogeneous and it is free from air bubbles and water, (ii) pouring it into the container, protecting it from dust and other particles in the atmosphere and allowing it to cool down in an temperature controlled atmosphere for a given time period, (iii) placing it along with the transfer dish in the water bath at a given temperature, filling the transfer dish with water from the water bath, placing the sample in it and taking it to the penetration apparatus platform, (iv) cleaning the needle with a liquid chemical compound, drying it and loading it with the weight, (v) adjusting the needle for making contact with the surface of the sample, (vi) taking the pointer of the measure indicator to zero, (vii) releasing the needle for a fixed time, (viii) adjusting the penetration apparatus for measuring the distance penetrated, (ix) making a fixed number of readings at different points on the surface of the sample and, finally, after the completion of every test, (x) returning the sample and the transfer dish to the water bath, cleaning the needle with a liquid chemical compound and drying it back. The measuring process in laboratory takes 4-5 hours, plus a production plan, also called crude oil structure, which takes 2-3 days. Besides, for achieving its optimum value, typically 4-5 laboratory measurements are needed.

Several methods for estimating properties of crude oil blends have been developed, based for example on Nuclear Magnetic Resonance (NMR) characterization data, Near Infrared (NIR) spectroscopy or other measurement techniques dependent on complex equipment, together with computerized, numerical techniques. However, data needed by these methods are not usually available in regular crude oil assays, being therefore necessary to continuously perform complex laboratory measurements. Examples of such methods are disclosed for instance in US6477516B1 and WO2008/135411A1. Similarly, Jaimes et al. describe in "Prediction of the Penetration Grade and Softening Point of Vacuum Residues and Asphalts by Nuclear Magnetic Resonance and Chemometric Methods", Energy Fuels 2019, 33, 6264-6272, a prediction model of the penetration grade and the softening point of vacuum residues (VRs) and pavement asphalts using computerized, numerical techniques. However, the proposed method is highly dependent on the structural data obtained with proton nuclear magnetic resonance (H NMR) and relaxometry data obtained via low-field nuclear magnetic resonance (LF NMR). Therefore, it needs NMR spectroscopy equipment. In addition, it requires performing specific measurements of the penetration grade and the softening point of every sample used for constructing the prediction models by means of standard laboratory analytical methods and equipment.

A method for predicting asphalt properties using measurements of kinematic viscosity of an asphalt fraction and information of the slate of crudes is disclosed in US2014180650A1. Faranda et al. have described in "Neural software to estimate vacuum residue quality", PTQ, Autumn 2002, a method for predicting the penetration value of bitumens (asphalt operation mode) using a computerized, numerical technique based on a neural network. However, it needs performing specific penetration measurements in laboratory very frequently (3-4 times a day).

In sum, several methods have been found to be capable of correlating characteristics of crude oils with properties to be predicted of processed crude oils or crude oil blends deriving therefrom. However, the reported methods, in particular those aiming at predicting the penetration value of a crude oil blend, are strongly dependent on regularly performing specific laboratory analysis.

Therefore, there is a need for developing a less costly, less time-consuming and less laboratory equipment dependent, in sum, a more efficient method for measuring or predicting the penetration value of the residue of the vacuum distillation unit at any given moment.

## DESCRIPTION OF THE INVENTION

The computer-implemented method and system for monitoring an oil refinery plant described in the present invention intends to solve the shortcomings of prior-art methods and systems therefor. The method predicts the penetration value of the residue of the vacuum distillation unit of the oil refinery plant. The method is implemented in a system that can be directly integrated into an oil refinery production plant. The prediction is performed online. This means that the prediction is performed substantially continuously, for example every few minutes. The prediction is performed from current production data of the oil refinery and from historic production data thereof. Current production data include production control operational data, laboratory data (such as data derived from analytical experiments performed in laboratory) and crude oil data (production scheduling data). Historic production data of the oil refinery includes historic data of the penetration value of the residue of the vacuum distillation unit.

The method and system do not need to perform new laboratory measurements or take new laboratory samples, nor use new measurement equipment (such as laboratory equipment, analyzers or sensors), with respect to measurements and equipment conventionally used in the plant. Rather, the method and system have the capability of using the regularly available production plant information, including production scheduling/planning information (that can be considered medium term varying information), laboratory analysis information (that can be considered low-to-medium term varying information) and real-time, production control operational information (rapidly varying information) of the plant. In the context of the present invention, the term "real time" refers to a time comprised within a range varying between 0.1 second and 30 minutes, such as range varying between 1 second and 30 minutes, or a range varying between 1 second and 15 minutes, or a range varying between 1 second and 10 minutes, or a range varying between 1 second and 5 minutes, or a range varying between 1 second and 2 minutes. One skilled in the art will understand that the evolution of technology may enable to reduce the minimum value of 1 second to a smaller value, such as 0.5 seconds, or 0.1 seconds.

A first aspect of the invention relates to a computer-implemented method for monitoring an oil refinery plant according to claim 1.

In embodiments of the invention, the production information of the oil refinery plant is processed as follows: independently processing the production scheduling information, the laboratory analysis information and the production control operational information; jointly processing the independently processed production information, thus constructing a principal dataset comprising a plurality of data samples.

In embodiments of the invention, the stage of jointly processing the independently processed production information, comprises: calculating a response time between information being input data to the vacuum distillation unit and output data therefrom, the input data comprising at least a flash zone temperature of the vacuum distillation unit, the output data comprising a real value of the penetration value of the residue of the vacuum distillation unit; averaging the data comprised in the production control operational information to be used for obtaining a prediction value, according to a time window determined by said response time and to a sampling time of the real value of the penetration value of the residue of the vacuum distillation unit; and joining the independently processed production information including the averaged data corresponding to the production control operational information source, thus constructing a principal dataset comprising a plurality of data samples.

In embodiments of the invention, the production scheduling information represents qualitative information about the crude oils inputted to the oil refinery as raw material, said qualitative information being the names of the crude oils and their percentages.

In embodiments of the invention, the at least one crude characterization parameter included in the laboratory analysis information is one of: amount of crude oil Sulphur (%w), amount of atmospheric residue yield (%v or %w), amount of vacuum residue asphaltenes (%w) and amount of heavy diesel yield (%v or %w).

In embodiments of the invention, the at least one temperature parameter of the vacuum distillation unit is a temperature related to the main distillation column of the vacuum distillation unit, and the at least one flow rate parameter of the vacuum distillation unit is the load to the vacuum distillation unit. The temperature related to the main distillation column of the vacuum distillation unit is preferably the flash zone temperature of the vacuum distillation unit.

In embodiments of the invention, the production control operational information further includes at least one pressure parameter of the vacuum distillation unit, said at least one pressure parameter of the vacuum distillation unit being preferably the flash zone column pressure in the vacuum distillation unit.

In embodiments of the invention, the numerical algorithm based on machine learning techniques comprises the following stacking regression models: a first regression model applying adaptive filtering techniques for catching rapidly varying dynamics information contained within the production control operational information; a second regression model applying neural network based regression techniques for catching quasi-static information contained within the laboratory analysis information; and a third regression model applying gradient boosting based regression techniques for catching slowly varying information contained within the production scheduling information.

A second aspect of the invention relates to a system -also referred to as soft sensor- for monitoring an oil refinery plant, according to claim 10.

In embodiments of the invention, the system further comprises: means for collecting said information of the oil refinery plant; means for processing the production scheduling information; means for processing the laboratory analysis information; means for processing the production control operational information; means for jointly processing the independently processed production information, thus constructing a principal dataset comprising a plurality of data samples.

In embodiments of the invention, the system further comprises storage means for storing the production information of the oil refinery plant, the predictive model and corresponding model evaluation metrics, the plurality of data samples of the principal dataset and the obtained prediction value.

In embodiments of the invention, the system further comprises means for displaying at least the obtained prediction value, said means being one of a computer monitor or a laptop computer screen, or a mobile phone screen, providing a graphical user interface.

The system may be embodied by a single, common computer system, or embodied by several, connected computer systems.

A third aspect of the invention relates to a computer program product comprising computer program instructions/code for performing the method of the first aspect of the invention.

A fourth aspect of the invention relates to a computer-readable memory/medium that stores program instructions/code for performing the method of the first aspect of the invention.

A full system that can be directly integrated into an oil refinery production plant for online prediction (monitoring) of the penetration value of the residue of the vacuum distillation unit, has been disclosed. It does not need to incorporate either new measurements -of new samples- nor to use new measurement equipment -laboratory equipment, analyzers or sensors). Rather, it uses the regularly available production plant information, including production scheduling/planning information, laboratory analysis information and real-time, production control operational information. In other words, it uses static information, medium-to-long term varying information and rapidly varying information of the plant.

Thanks to the method and system of the present invention, the performance of the vacuum distillation unit of an oil refinery is improved by achieving an optimum value of the penetration value of the residue of the vacuum distillation unit in a shortest time. Therefore, the performance of the coker unit of the oil refinery is also improved, as well as the performance of the oil refinery.

Furthermore, a reduction in the number of regularly performed needle penetration method-based laboratory measurements of the penetration value of the residue of the vacuum distillation unit is achieved. This reduction is possible thanks to the reliable predictor (soft sensor) that continuously provides penetration predictions.

Additional advantages and features of the invention will become apparent from the detailed description that follows and will be particularly pointed out in the appended claims.

## BRIEF DESCRIPTION OF THE DRAWINGS

To complete the description and in order to provide a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

Figure 1 shows a general diagram of an oil refinery, in which typically crude oils are inputted as raw material and different shares of petroleum products, such as refined products and transformed sub-products, are outputted.

Figure 2 schematically shows a crude oil distillation unit, which distills the incoming crude oil into various atmospheric distillate fractions, and whose atmospheric residue is further processed in other units, typically in a so called vacuum distillation unit.

Figure 3 schematically shows a vacuum distillation unit, in which residue oil coming from a crude oil distillation unit is further distilled. Among other products, it outputs vacuum residue, which is further processed in other units, typically in the so-called coker unit.

Figure 4 schematically shows a coker unit which converts vacuum residue coming from the vacuum distillation unit into gases, coker sub-products and petroleum coke.

Figure 5A shows a block diagram representing a system for on-line monitoring an oil refinery, comprising a computer system, according to an embodiment of the present invention.

Figures 5B to 5D show different embodiments of the system of the invention, in which the computer system is implemented by a single, physical computer system (Figure 5B), or by a single, virtual computer system (Figure 5C), or by a physical or virtual computer network (Figure 5D).

Figures 5E to 5G show different embodiments of the system of the invention, in which the computer system is implemented by a single, virtual computer system and the computer monitor is a laptop computer screen (Figure 5E), a tablet computer screen (Figure 5F) or a mobile phone screen (Figure 5G).

Figure 6 shows a flow diagram representing the method for on-line monitoring an oil refinery according to an embodiment of the invention.

Figure 7 shows a flow diagram including stages of data fetching and gathering for getting data from different sources: production scheduling/planning information source, laboratory analysis information source and real-time, production control operational information source, according to embodiments of the invention.

Figure 8 shows a flow diagram including stages of data processing according to embodiments of the invention.

Figure 9 shows a flow diagram including stages for identifying the operational status, either running or stopped, of a vacuum distillation unit of an oil refinery, according to embodiments of the invention.

Figure 10 shows a flow diagram including stages for joint data processing according to embodiments of the invention.

Figure 11 shows a flow diagram including stages for constructing/upgrading a predictive model and calculating model evaluation metrics, according to embodiments of the invention.

Figure 12 shows a flow diagram including stages for determining the penetration value of vacuum distillation residue, according to embodiments of the invention.

Figure 13 shows a flow diagram including stages for storing datasets, the predictive model and corresponding model evaluation metrics, and the prediction determined by the predictive model, according to embodiments of the invention.

Figure 14 shows a flow diagram including stages for constructing and rendering a GUI, according to embodiments of the invention.

## DESCRIPTION OF A WAY OF CARRYING OUT THE INVENTION

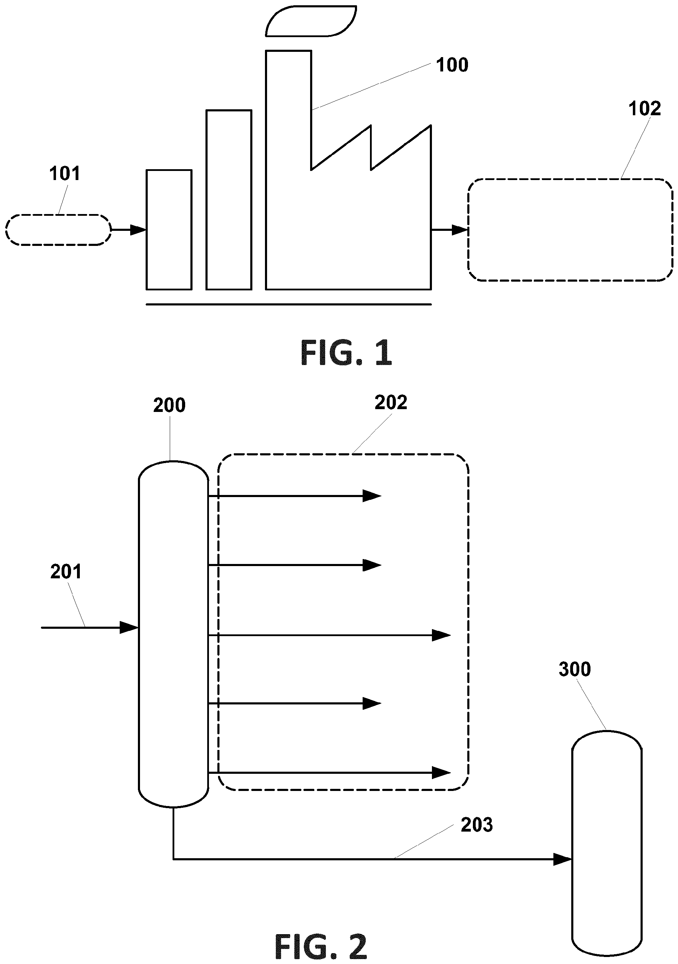

Figure 1 schematically shows a general diagram of an oil refinery 100, where typically crude oils 101 are inputted as raw material and different shares of petroleum products 102 are outputted. Output petroleum products 102 are typically refined product or transformed sub-products. They are usually grouped into four categories: light distillates (LPG, gasoline, naphtha); middle distillates (kerosene, jet fuel, diesel); heavy distillates; and residue (heavy fuel oil, lubricating oils, wax, asphalt).

The present invention relates in particular to the following units of an oil refinery: the crude oil distillation unit, the vacuum distillation unit and the coker unit. Figure 2 shows a crude oil distillation unit 200, also called atmospheric distillation unit, which distills the incoming crude oil 201 into various atmospheric distillate fractions 202, typically light naphtha, heavy naphtha, jet fuel and/or kerosene, diesel oil and atmospheric gas oil. The crude oil distillation unit 200 produces atmospheric residue 203, which usually comes out from the bottom of the unit 200. The atmospheric residue 203 is further processed in other units, usually in the vacuum distillation unit 300. In an oil refinery there may be more than one crude oil distillation units 200. For example, there may be two crude oil distillation units, generally represented under reference 200 in Figure 2.

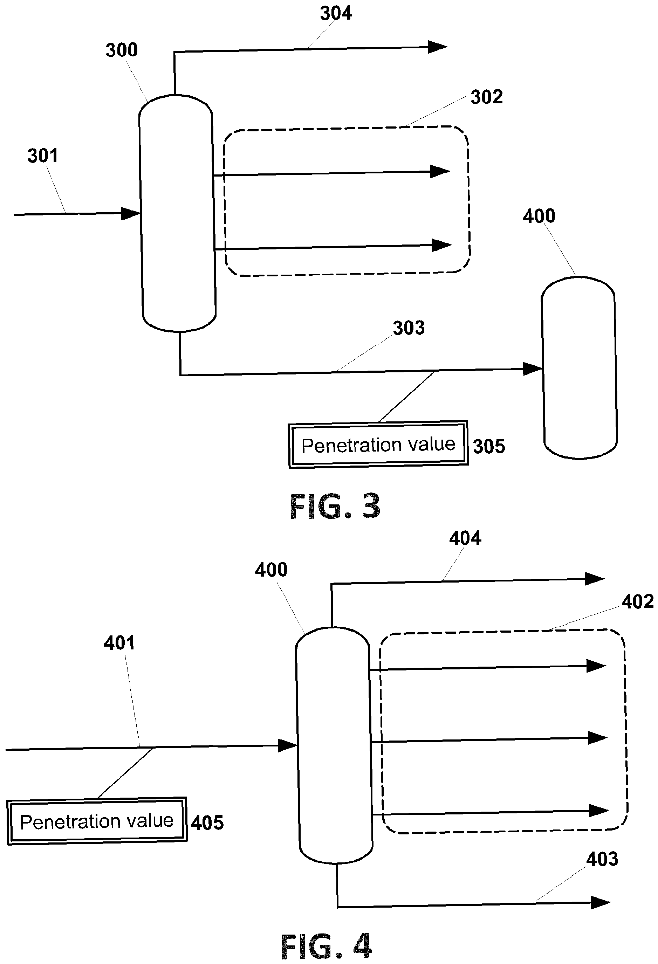

Figure 3 shows a vacuum distillation unit 300, which is an additional, intermediate distillation unit where atmospheric residue 301 coming from the bottom of crude oil distillation unit 200 is further distilled. In this process, vacuum distillate fractions 302, such as light vacuum gas oil and heavy vacuum gas oil, are obtained. Non-condensable gases 304 are evacuated - typically from the top of unit 300, and vacuum residue 303 is outputted, typically from the bottom of unit 300. Vacuum residue 303 is further processed in other units, typically in the coker unit 400. Figure 4 shows a coker unit 400, in which the vacuum residue 401 coming from the vacuum distillation unit 300 is converted into several products: petroleum coke 403, which is the coker main product; coker sub-products 402, such as coker naphtha, light coker gas oil and heavy cooker gas oil; and low molecular weight hydrocarbon gases 404.

The method and the system of the present invention refer to the vacuum residue 303 obtained at the vacuum distillation unit 300 and processed at the coker unit 400. In particular, a method and system have been developed, for the online monitoring of the penetration value 305 of the vacuum residue 303 in the vacuum distillation unit 300. The penetration value 305 of the vacuum residue 303 is a critical production operation parameter in oil refining processes. The method and system of the invention are also referred to as a soft sensor. The method and system are valid for any production operation mode (asphalt and non-asphalt production). This means that it is valid for the whole range of penetration values (up to tenths of a millimeter).

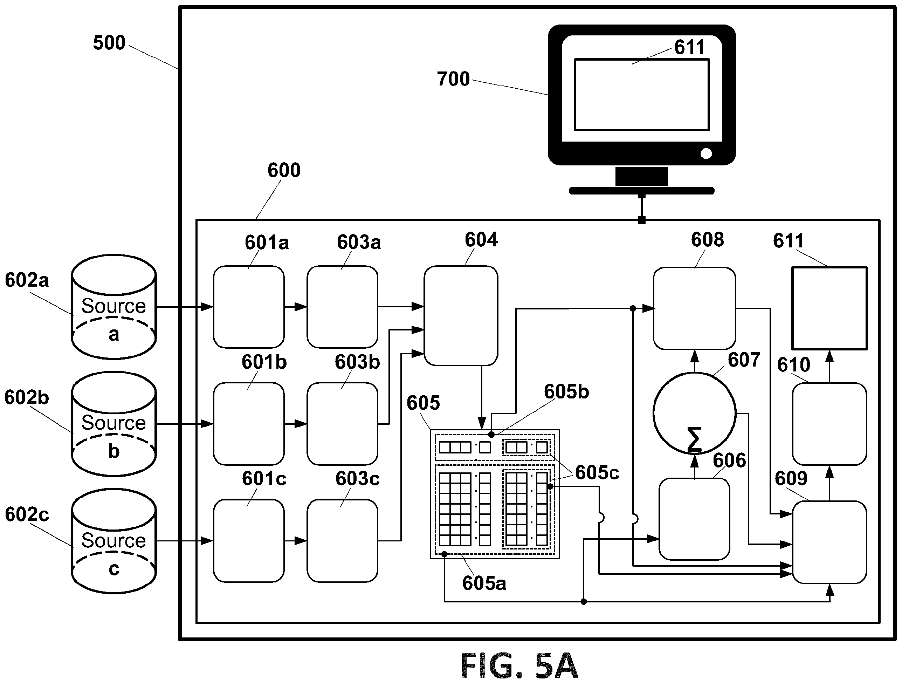

A system for implementing the method for the online monitoring of the penetration value 305 of the vacuum residue 303 (from now on, the penetration value) is schematically represented in Figure 5A. The system implements a soft sensor 500. In other words, it is a soft sensor-based online monitoring system 500 for oil refineries 100. System 500 is formed by a computer system 600 and an input/output interface 700, for example implemented by a computer monitor, which displays graphical user interface 611. Input/output interface 700 permits to monitor in real-time the prediction of penetration value and one or more production variables (parameters) relevant in relation to the penetration value. Monitoring one or more of such variables contributes to correctly interpret the predictions. Figure 5A shows a block diagram of computer system 600, whose main elements or blocks are described next.

The computer system 600 includes first, second and third data fetching and gathering means 601a, 601b and 601c, implemented by respective pieces of computer code in charge of fetching and gathering information from plant production information sources 602a, 602b and 602c, respectively. The information stored in these sources is data inputted into or outputted from the oil refinery 100. This information may be used to build a prediction model for the penetration value.

Information source 602a represents production scheduling/planning information. Usually, data collected from information source 602a represents qualitative information about the crude oils 101 inputted to the oil refinery 100 as raw material. In other words, data collected from source 602a refers to variables or parameters qualitatively defining the crude oils. Preferably, data collected from source 602a comprises the names of the specific crudes (or types thereof) and their percentages (above a total amount represented as 100%) as defined in the actual production plan. Non-limiting examples of types of crude that may be collected from source 602a are: AMENAM, AGBAMI, BASRAH, BASRAH PESADO, BONGA, CASTILLA, ESSIDER, FORTIES, IRAN PESADO, KIRKUK, MAYA, MORICHAL, PEREGRINO, RONCADOR HEAVY, SANKOFA and URAL PRIMORSK. In order to build a prediction model for the penetration value, a production plan including one or more types of crude from source 602a -and its/their percentages- is used. The crudes and percentages of this plan will be used. The amount of each crude varies between 0% and 100%, the sum of all the crudes included in a production plan being substantially 100%. Source 602a is updated at a medium rate frequency, such as every few days, for example every 1-3 days, such as once per day, or every two days, or every three days.

Information source 602b represents laboratory analysis information. Data collected from source 602b includes information corresponding to historical values of penetration value 305 of the vacuum residue 303 of the vacuum distillation unit 300. In particular, the penetration value 305 represents output data from the vacuum distillation unit 300. Data collected from source 602b also includes crude oil characterization features that represent quantitative information about the crude oils 101 inputted to the oil refinery 100 as raw material. Crude oil characterization features may be obtained experimentally (e.g. from measurements made in laboratory), from databases, from physical models or from combinations of the former methodologies. Non-limiting examples of crude oil characterization features that may be collected from source 602b are: crude oil density, amount of crude oil sulphur (%w), amount of naphtha yield (%w or %v), amount of kerosene yield (%w or %v), amount of light diesel yield (%w or %v), amount of heavy diesel yield (%w or %v), amount of gasoil yield (%w or %v), amount of atmospheric residue yield (%w or %v), amount of light gasoil yield (%w or %v), amount of heavy gasoil yield (%w or %v), amount of vacuum residue yield (%w or %v), atmospheric residue density, vacuum residue density, amount of atmospheric residue Sulphur (%w), amount of vacuum residue Sulphur (%w), amount of atmospheric residue CCR (Conradson carbon residue) (%w), amount of vacuum residue CCR (Conradson carbon residue) (%w), amount of atmospheric residue asphaltenes (%w), amount of vacuum residue asphaltenes (%w), vacuum residue penetration (mm/10) and atmospheric residue penetration (mm/10). In addition to historical values of penetration value, in order to build a prediction model for the penetration value, at least the amount of vacuum residue asphaltenes (%w) is preferably used from source 602b. In a more preferred embodiment, at least one additional crude oil characterization feature is used, selected from the following ones: amount of crude oil sulphur (%w) and amount of atmospheric residue yield (%w or %v). In a still more preferred embodiment, the crude oil characterization features used are the amount of vacuum residue asphaltenes (%w), the amount of crude oil sulphur (%w), the amount of atmospheric residue yield (%w or %v) and the amount of heavy diesel yield (%w or %v). Optionally, additional crude oil characterization features may be collected. Source 602b is updated at a low-medium frequency: while most crude oil characterization features are updated at a low rate frequency, such as every few months, for example once every 3-6 months, historical values of penetration value 305 are upgraded at a faster frequency, such as between every several hours and every few days.

Information source 602c represents real-time production control operational information obtained at different points in the oil refinery plant. Data collected from source 602c usually represents the capture of a plurality of sensors, actuators and/or controllers used for controlling operational variables of the oil refinery plant, such as temperature, pressure and/or flow rate (load). In order to build a prediction model for the penetration value, parameters of temperature, pressure and flow rate (load) measured at the vacuum distillation unit, at any unit affecting the crude-vacuum-coker line, or at any other unit in the oil refinery plant, may be used. Non-limiting examples of real-time production control operational information that may be collected from source 602c are:

Regarding load (flow rate): load to vacuum unit, loads from tanks in vacuum unit, vacuum unit loads coming from previous distillation units (such as loads from crude units), vacuum unit lane loads (such as loads to different load lanes of vacuum unit), overloads in vacuum unit (such as overloads to tanks in vacuum unit), production of residues and products in vacuum unit (such as total residue production, bottom residues of the main distillation column and washing heavy gas oil, in vacuum unit), production of vapours and gases in vacuum unit (such as bottom depletion vapour of the main distillation column, ejector vapour from the different ejectors at the unit, incondensable gases to furnaces, in vacuum unit) and flow-rates and loads from other streams or units upstream or downstream of the vacuum unit (such as loads to the crude units, loads to mixing tanks, atmospheric gas oil reflow in crude units, stripping steam at the bottom of crude units, stripping steam in crude units, overloads to tanks in crude units).

Regarding pressure: load pressures in vacuum unit (such as load pressures from different tanks in vacuum unit), pressures from different zones of the vacuum unit (such as head pressure of the distillation column, flash zone column pressure, in the vacuum unit) and pressures from other streams or units upstream or downstream of the vacuum unit (such as flash zone pressure of the main distillation columns in crude units).

Regarding temperature: load temperatures in vacuum unit (such as load temperatures from the different heat exchangers in vacuum unit), temperatures from different zones of the vacuum unit, specially the ones related to the main distillation column (such as bottom distillation column temperature, bottom extraction temperature of the (main) distillation column, flash zone temperature, in the vacuum unit) and temperatures from other streams or units upstream or downstream of the vacuum unit (such as atmospheric gas oil output temperature in crude units, atmospheric gas oil extraction plates temperatures in crude units, flash temperature in crude units, atmospheric gas oil cold reflow temperature for the distillation column in crude units, various plates temperatures for the different distillations columns in crude units, specially the main distillation column of the crude units).

Preferably, at least one temperature parameter and at least one flow rate (load) parameter are used. The at least one temperature parameter is preferably a temperature measured in the vacuum unit, more preferably a temperature related to the main distillation column of the vacuum unit. Still more preferably, this temperature is the flash zone temperature in the vacuum unit. The at least one flow rate (load) parameter is preferably the load to vacuum unit. It is remarked that any other temperature parameter or flow rate (load) parameter which is respectively correlated with the flash zone temperature in the vacuum unit or with the load to vacuum unit, may be respectively used instead.

More preferably, at least one temperature parameter, at least one pressure parameter and at least one flow rate (load) parameter are used. The at least one temperature parameter is preferably a temperature measured in the vacuum unit, more preferably a temperature related to the main distillation column of the vacuum unit. Still more preferably, this temperature is the flash zone temperature in the vacuum unit (or a temperature parameter correlated thereto). The at least one flow rate (load) parameter is preferably the load to vacuum unit (or a flow rate (load) parameter correlated thereto). The at least one pressure parameter is preferably a load pressure in the vacuum unit, more preferably the flash zone column pressure in the vacuum unit (or a pressure parameter correlated thereto).

Other load, pressure and temperature parameters that may be additionally used for building a prediction model for the penetration value, are: load to vacuum unit, loads from tanks in vacuum unit, loads from crude units, loads to different load lanes of vacuum unit, overloads to tanks in vacuum unit, total residue production in vacuum unit, bottom residues of the main distillation column in vacuum unit, washing heavy gas oil in vacuum unit, bottom depletion vapour of the main distillation column in vacuum unit, ejector vapour from the different ejectors in vacuum unit, incondensable gases to furnaces in vacuum unit, loads to crude units, loads to mixing tanks, atmospheric gas oil reflow in crude units, stripping steam at the bottom of crude units, stripping steam in crude units, overloads to tanks in crude units; load pressures from different tanks in vacuum unit, head pressure of the distillation column in the vacuum unit, flash zone main column pressure in the vacuum unit, flash zone pressure of main distillation column in crude units; load temperature to vacuum unit, bottom column temperature in the vacuum unit, bottom main distillation column temperature in the vacuum unit, atmospheric gas oil output temperature in different crude units, atmospheric gas oil extraction plates temperatures in different crude units, various plates flash temperatures in different crude units, atmospheric gas oil extraction gradient flash temperature in different crude units, atmospheric gas oil cold reflow temperature for the distillation column in different crude units, various plates temperatures in the main distillation column in different crude units, bottom output temperature of different distillation columns in different crude units, flash zone temperature in the main distillation column in different crude units. In a particular embodiment, all available parameters of temperature, pressure and load (flow rate) of the crude-vacuum-coker line, are used for building the prediction model.

Source 602c is updated at a high rate frequency, such as every few seconds or minutes, for example once between 1 second and 30 minutes, or once between 1 second and 15 minutes, or once between 1 second and 10 minutes, or once between 1 second and 5 minutes, or once between 1 second and 2 minutes. By selecting the preferred variables (model features), an accurate and robust predictive model can be later obtained.

Computer system 600 also includes three first information processing means 603a, 603b and 603c, implemented by respective pieces of computer code in charge of independently processing the information collected separately, at their respective frequencies, by respective data fetching and gathering means 601a, 601b and 601c. The information processed separately by first information processing means 603a, 603b and 603c is then inputted into second information processing means 604. Information processing at blocks 603a, 603b, 603c includes removing eventual outliers and erroneous data from the corresponding gathered data and, if required, filling gaps and missing data in the respective source (production scheduling/planning information source 602a, laboratory analysis information source 602b and real-time, production control operational information source 602c). Second information processing means 604 is implemented by one or more pieces of computer code in charge of jointly processing the information processed independently at first information processing means 603a, 603b and 603c.

At the second processing means 604, a principal, merged input dataset 605 in the form of a time series is constructed by joining the data independently processed by first processing means 603a, 603b and 603c. In order to accomplish this, time synchronization techniques based on the sampling time of the output data (which is the production operation parameter to be predicted, that is to say, the penetration value 305 of the residue 303 of the vacuum distillation unit 300), collected/sampled/updated at a frequency rate of between every several hours and every few days), included in the data collected from source 602b and subsequently processed by processing means 603b, and the data corresponding to the real-time, production control operational information 602c represented by the previously processed (i.e. averaged) corresponding data obtained at block 603c, are applied. In other words, time synchronization is made between the samples of penetration value (collected from 602b) and averaged samples of production control operational variables (collected from 602c). Data originally coming from the production scheduling/planning information source 602a and other data (different from the penetration value) coming from the laboratory analysis information source 602b do not need to be considered for the time synchronization process because these data are static with respect to the penetration value 305, and therefore are already synchronized with respect to it.

Principal dataset 605 comprises different subsets in the form of a time series and arranged in a data frame format: a first, auxiliary, merged input dataset 605a (also referred to as first subset 605a) and a second, auxiliary, merged input dataset 605b (also referred to as second subset 605b). A production control operational variables dataset 605c (also referred to as third subset 605c) may also be comprised in principal dataset 605. These subsets 605a, 605b, 605c will be subsequently and correspondingly used for constructing a predictive model, executing predictions and optionally displaying production control operational variables at GUI 611 as will be described later.

Principal dataset 605 may be represented in the form of a matrix having R rows and K columns, R, K being natural numbers. There is one column per variable from sources 602a, 602b, 602c. If, for example, there are A different variables (parameters) in source 602a, B different variables (parameters) in source 602b and C different variables (parameters) in source 602c, then the number of columns K in the matrix is K = A + B + C. Regarding rows R, there is one row per temporal capture, including the captures collected at the most recent time instant, $t$. Temporal captures may be done, for example, every 15 minutes. In other words, the total number of rows R represents the historical data of sources 602a, 602b, 602c.

In particular, first subset 605a will be consumed by predictive model construction means 606, not yet explained in detail. First subset 605a includes the data required for training the predicting model. First subset 605a may be represented in the form of a matrix having K columns (one per variable or parameter) and as many rows $R_a$ as the number of available real samples of vacuum penetration taken at the laboratory at different time instants. In other words, in this case there are as many rows $R_a$ as historical, real samples of vacuum penetration. Because first subset 605a is used to train (construct) the model, the relevant data for training the model are the data collected at the time instants in which real vacuum penetration values have been obtained at laboratory. Therefore, every time a new sample of penetration value is made at the laboratory, a new row is added to this matrix, which implies that the predictive model can be retrained (reconstructed). Among the different elements in a new row (elements representing values of variables A, B and C), some of them may be updated with respect to the previous row (elements corresponding to variables that have been updated) and some of them may remain unaltered (elements corresponding to variables being updated at medium-low frequency). Data is synchronized according to the data synchronization process as previously explained, that is to say, time synchronization is made between the samples of penetration value taken at laboratory and averaged samples of production control operational variables.

In turn, second subset 605b will be consumed by prediction determination means 608, not yet explained in detail. Second subset 605b includes the last sample of the dataset made up by the variables used for performing the prediction, and is then added to the already stored data collection for retraining the model when required. Second subset 605b may be represented in the form of a matrix having a single row and K-1 columns (all variables or parameters are represented except the variable to be predicted, that is to say, the penetration of the vacuum residue). In other words, subset 605b is a vector of K-1 elements. Subset 605b is updated at high frequency. In fact, it is updated in real-time, that is to say, it is updated with a frequency varying between 0.1 second and 30 minutes, such as every 15 minutes or every 5 minutes, or even more frequently. If, for example, a new prediction is made every 15 minutes, this second subset 605b is updated every 15 minutes. Considering two consecutive subsets 605b, not all the values of their elements are modified with respect to a previous subset 605b. Among the elements comprised in a subset 605b, those which typically may change with respect to the previous subset 605b (for example built 15 minutes before) are the production control operational information (from source 602c), because parameters obtained from source 602c are updated at a high rate frequency (for example once between 1 second and 30 minutes, such as every 15 minutes or even every minute). Other elements comprised in subset 605b that may also change, but less often, with respect to the previous subset 605b are: production scheduling information (from source 602a), but they will only change if there is a new schedule, which may happen at a medium rate frequency (for example every 1-3 days, such as once per day); and laboratory analysis information (from source 602b) other than the penetration value, but they are updated at a low rate frequency, such as every few months, for example once every 3-6 months.

Finally, third subset 605c may be consumed by visualization construction means 610, not yet explained in detail, and therefore displaying means 700. Third subset 605c may be represented in the form of a matrix having R rows (available historical data) and as many columns as variables (parameters) to be displayed on a GUI. In a particular embodiment, only a few variables of the production control operation information (from source 602c) are comprised in subset 605c and therefore available for visualization. Non-limiting examples of variables to be comprised in subset 605c are: the flash temperature of the vacuum distillation unit 300, the pressure of the vacuum distillation unit 300 at the flash region and the load to vacuum distillation unit 300. In a more particular embodiment, only one of these variables may be displayed, for example the flash temperature of the vacuum distillation unit 300, the pressure of the vacuum distillation unit 300 at the flash region or the load to vacuum distillation unit 300. In other particular embodiments, two of them are displayed, or the three of them. Also, other variables different from these ones may be displayed. Like in subset 605a, in subset 605c there is one row per temporal capture, including the captures collected at the most recent time instant, t. Temporal captures may be done, for example, every 15 minutes.

Computer system 600 also comprises one or more pieces of computer code for implementing different blocks: means 606 for construing a predictive model 607 for predicting the penetration value 305 of residue 303 (vacuum residue) -as illustrated for example in Figures 3 and 4- and calculating model evaluation metrics; means 608 for determining a prediction for the penetration value 305 by executing predictive model 607, and optionally, means 610 for visualization construction, for constructing and rendering a graphical user interface 611 showing for example the online, time evolution of the prediction value of the penetration 305 of the vacuum residue determined by the predictive model 607, the predictive model evaluation metrics, and the online time evolution of at least some of the production control operational variables 605c required for determining the optimum value of the penetration value of the residue of the vacuum distillation unit in the shortest time. Details of these blocks 606, 608, 610 will be described later together with corresponding data flows.

Computer system 600 also has information storage means 609, such as one or more pieces of memory, for storing different information involved in the different blocks of the computer system 600, such as the previously determined prediction for the penetration value 305, a time stamp of a prediction time step t, where t represents current time step, values of the data making up first and second subsets 605a, 605b for the associated prediction moment (time t of the dataset 605b with which the prediction is made), the predictive model 607 and corresponding model evaluation metrics, and values of production control operational variables dataset 605c, required for determining the optimum value of the penetration value 305 in a shortest time or for correctly interpreting the predictions.

In every execution cycle of computer system 600, datasets 605 (including 605a, 605b, 605c), predictive model 607 and corresponding model evaluation metrics, prediction of penetration value 305, storage of predictive model 607 and all the above data, and, eventual construction and rendering process of graphical user interface 611, according to every new production information generated in the plant, are automatically upgraded. However, this automatic upgrading may be ignored when an operation stop is detected for the vacuum distillation unit of the oil refinery.

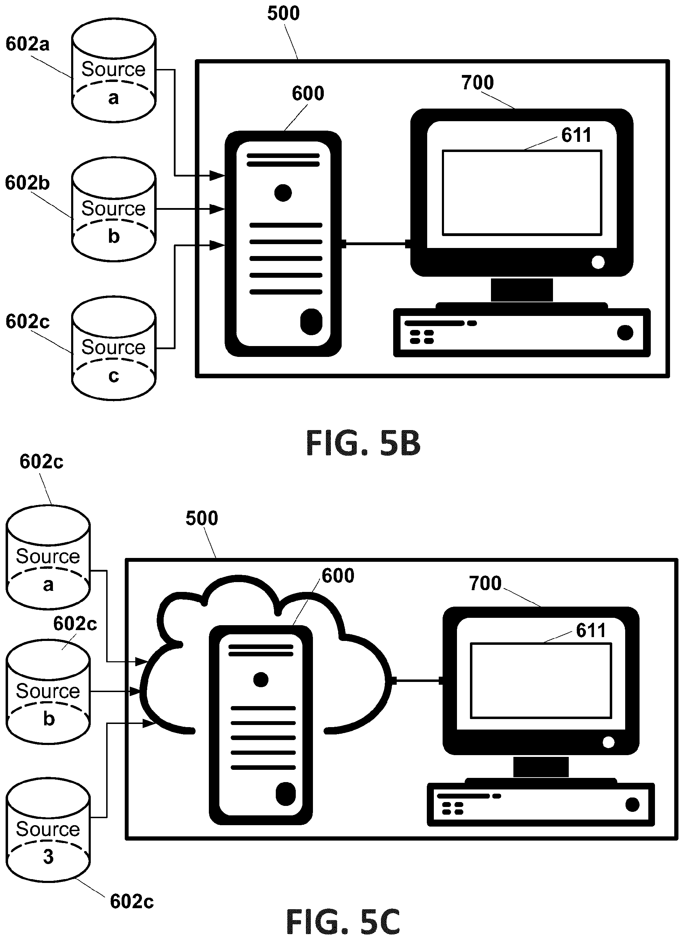

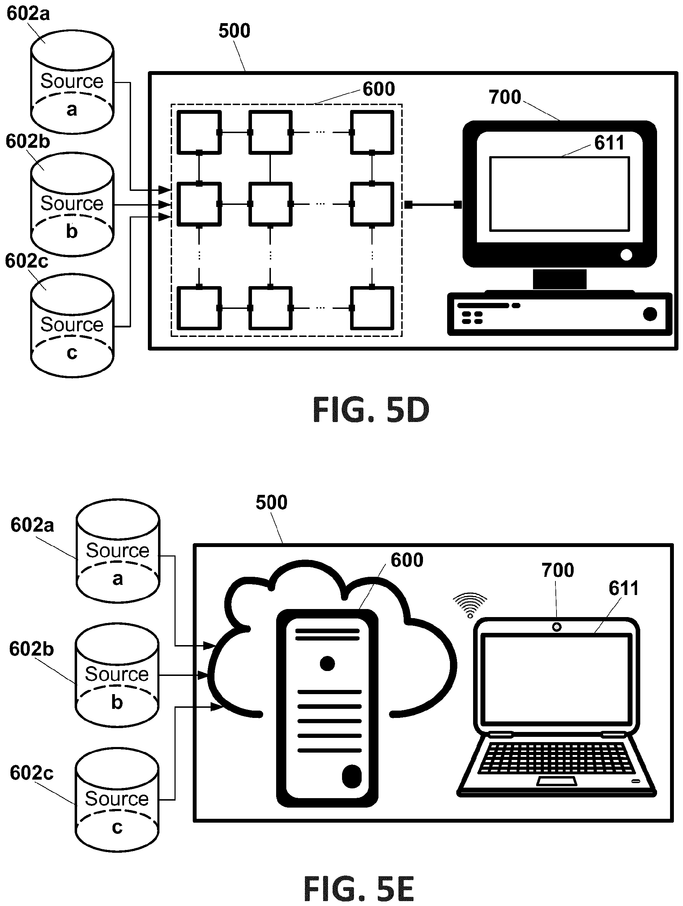

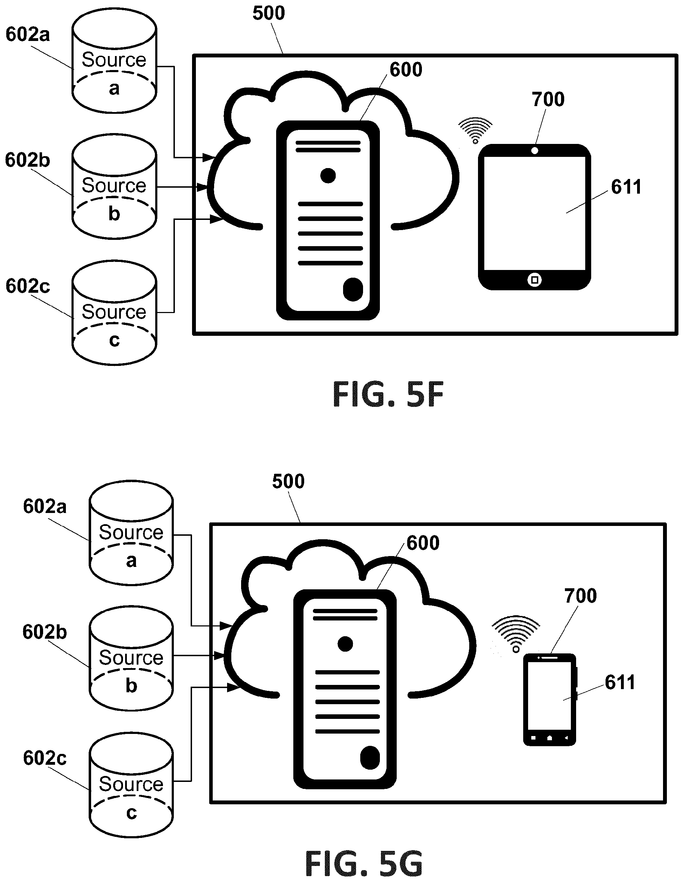

System 500 may be embodied following different implementations. Figures 5B to 5G illustrate exemplary implementations of system 500. In Figure 5B, the computer system 600 is a single, physical (hardware) computer system, such as a personal computer having a CPU (single processor or multi-core processor) assembled in a single hardware equipment. In Figure 5C, the computer system 600 is a single, virtual computer system, such as a server located in the cloud. In Figure 5D, the computer system 600 is a computer network generally comprising m x n separate processors, either physical (for example deployed within the plant under monitoring) or virtual (for example located in the cloud and distributed along different separate processors or servers). In examples 5B to 5D the computer or group of computers 600 is connected to a computer monitor 700, acting as GUI 611. In Figure 5E, the computer system 600 is a single, virtual computer system and the computer monitor 700 is a laptop computer screen. In Figure 5F, the computer system 600 is a single, virtual computer system and the computer monitor 700 is a tablet computer screen. In Figure 5G, the computer system 600 is a single, virtual computer system and the computer monitor 700 is a mobile phone screen. The connection between the computer system 600 and the monitor 700 acting as GUI may be done either wired or wirelessly. In any one of the possible implementations of system 500, the computer system comprises processing means, such as one or more processors, having embedded computer program code for executing the method for the online monitoring of an oil refinery of the present invention. The different blocks identified in Figure 5A -first, second and third data fetching and gathering means 601a-601c, three first information processing means 603a-603c, second information processing means 604, predictive model construction means 606, prediction determination means 608, information storage means 609 and visualization construction means 610- may be implemented in one or several processing means, such as one or more processors. One skilled in the art will understand that a single processor may embed several of these means, or alternatively a single means may be embedded in a plurality of distributed processors, or any other combination thereof.

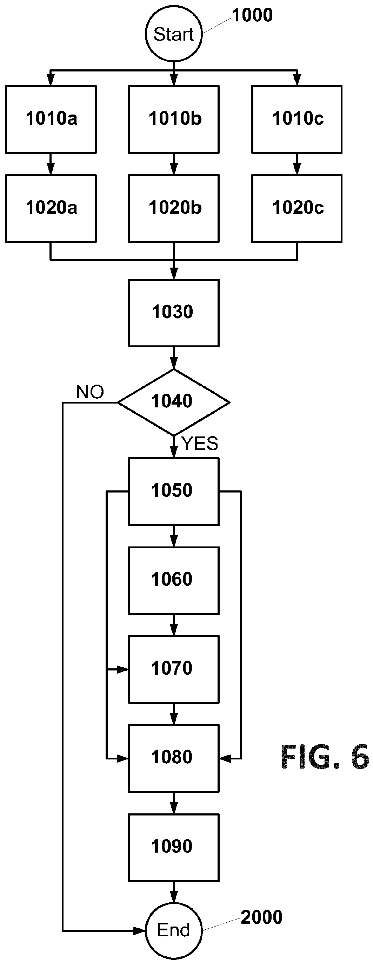

Figures 6 to 14 show different flow diagrams representing different stages of the computer-implemented method for predicting the penetration value of the residue of the vacuum distillation unit.

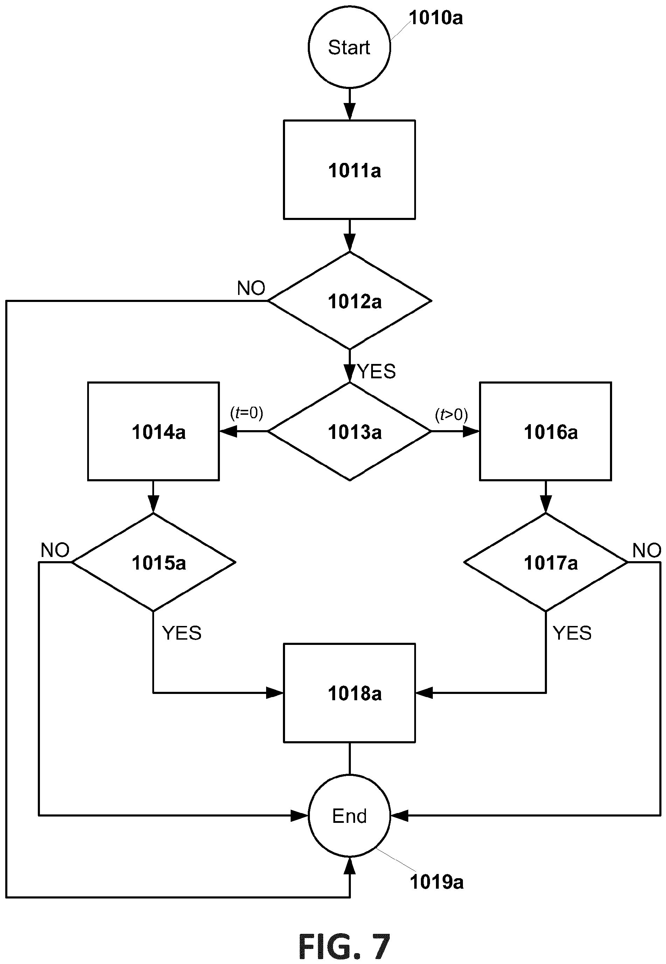

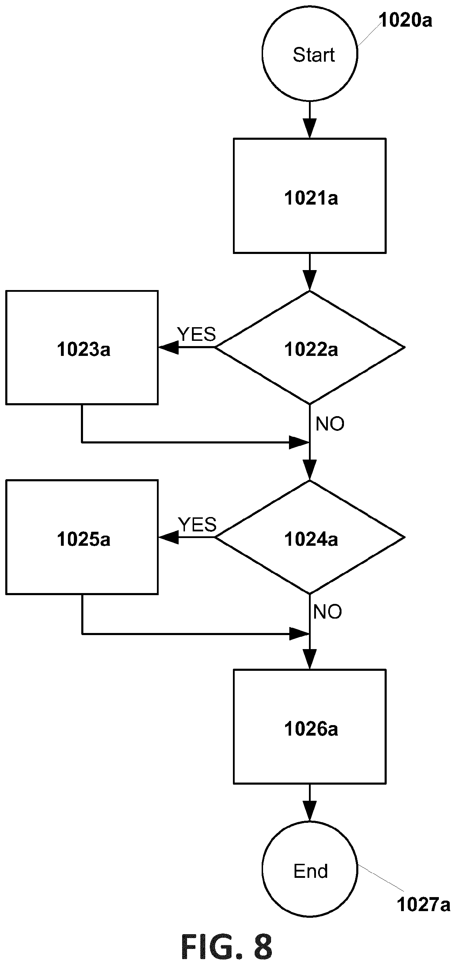

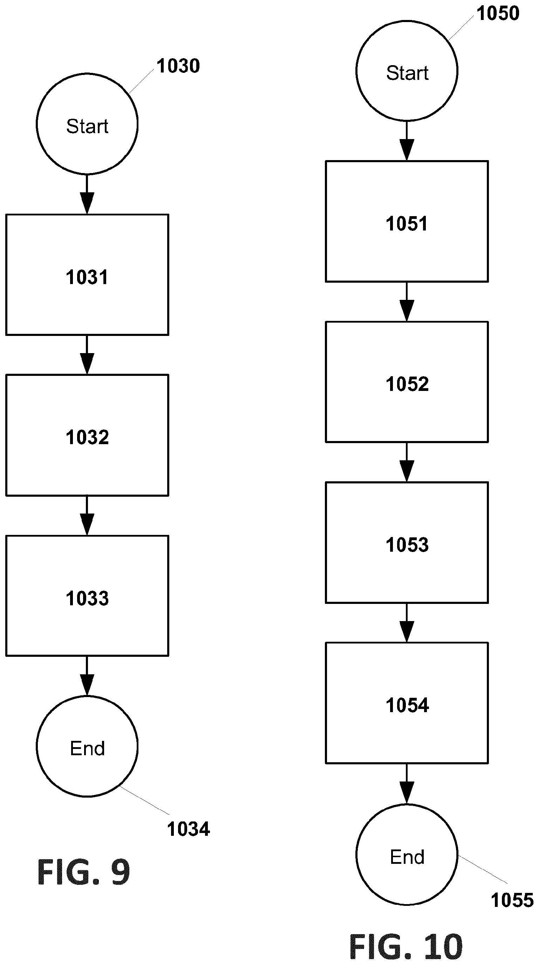

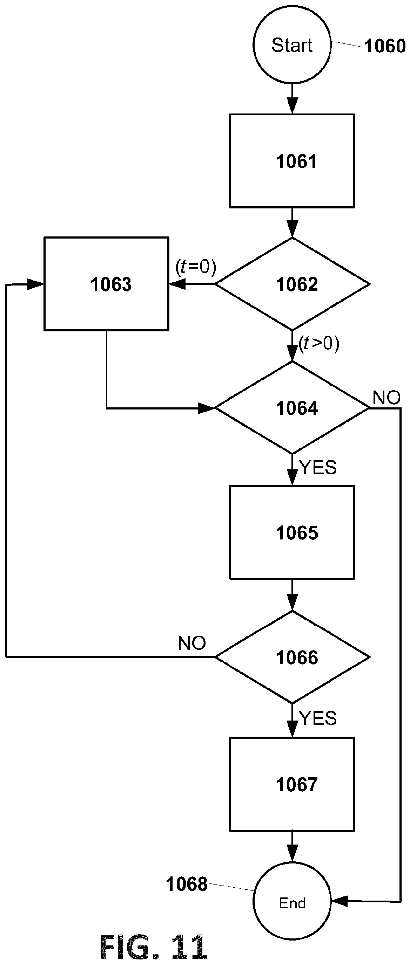

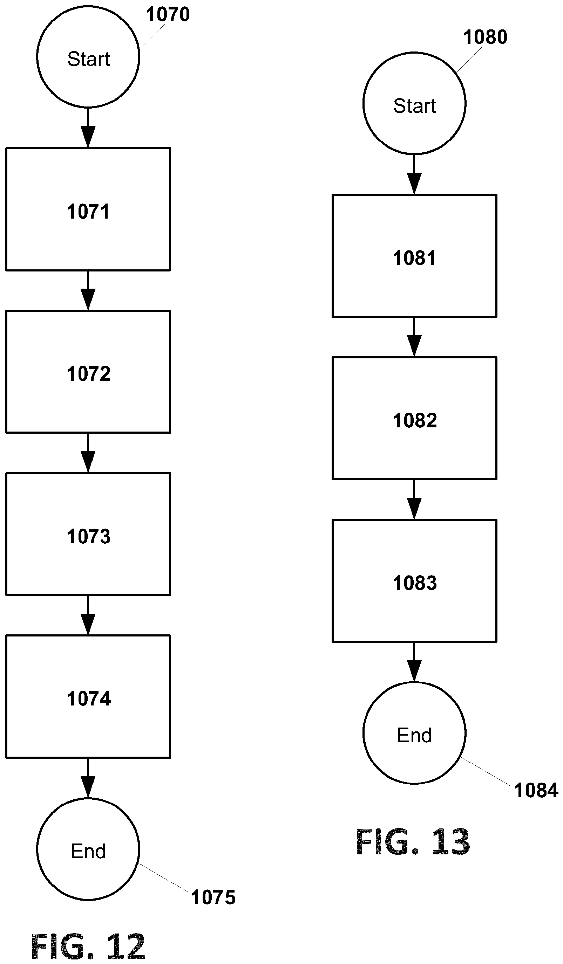

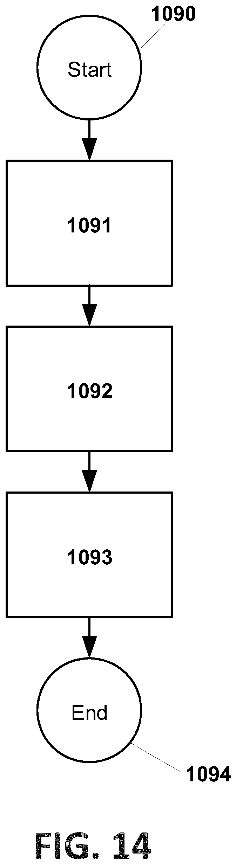

Figure 6 shows a general flow diagram representing the method of the invention through stages 1000 to 2000. These stages are explained in detail with further reference to figures 7-14. The execution of the method starts at time step t (stage 1000), where data fetching and gathering means 601a, 601b and 601c establish a secure connection to information sources 602a, 602b and 602c, respectively (stages 1010a, 1010b and 1010c) and collect data, in real-time, therefrom. For example, if production control operational parameters are updated at source 602c every minute, the values of these parameters can be collected with substantially no delay (for example, a delay of 1 second) if source 602c is interrogated every second. This is explained in detail regarding Figure 7. The information collected at stages 1010a, 1010b and 1010c is processed by first information processing means 603a, 603b and 603c, independently, at stages 1020a, 1020b and 1020c, respectively. This is explained in detail regarding Figure 8. Then, a task of identifying the operational status of the of vacuum distillation unit 300 of oil refinery 100 is accomplished (stage 1030). The operational status may be running (in operation) or stopped. This is explained in detail regarding Figure 9. Next, a task of checking whether the stop of a unit is detected, is accomplished (stage 1040): if a unit stop is detected ("NO" in stage 1040), then the execution of all subsequent stages and associated tasks for current time step t is ignored and the execution of the method for time step t+1 is started (stage 1000), where t+1 represents next time step; otherwise ("YES" in stage 1040), then the execution of tasks continues. The information independently provided by stages 1020a, 1020b and 1020c is further processed, jointly, by second information processing means 604 (stage 1050), wherein a principal, merged input dataset 605 is built. This is explained in detail regarding Figure 10. Dataset 605 mainly includes two subsets: the already disclosed first subset 605a and second subset 605b, in the form of a time series. Dataset 605 may also include the already disclosed third subset 605c. These subsets represent the main datasets to be used by subsequent stages for the construction of a predictive model (stage 1060), performing a prediction (stage 1070), storage (stage 1080) and displaying (stage 1090), as already explained. First subset 605a includes the data required for training the predicting model (block 606 in Figure 5A). Second subset 605b includes the data required for performing a prediction (block 608 in Figure 5A). Second subset 605b includes the last sample of the dataset made up by the variables used for performing the prediction. Once the prediction is made, this sample is then added to the already stored data collection for retraining the model when required. Third subset 605c may be used for later visualization and displaying. Thus, at stage 1060 the first subset 605a obtained at stage 1050 is used for constructing predictive model 607 and for calculating a set of corresponding model evaluation metrics. Constructing the predictive model 607 should be understood as either constructing it for the first time if ($t=0$) or upgrading it, if ($t>0$). This is explained in detail regarding Figure 11. At stage 1070 the second subset 605b, also obtained at stage 1050, is used for determining a prediction for the penetration value 305 by executing predictive model 607 obtained at previous stage 1060. This is explained in detail regarding Figure 12. Then, at stage 1080 data is stored. In particular, datasets 605a, 605b and 605c, predictive model 607 and corresponding model evaluation metrics and predicted penetration value may be stored, preferably in a persistent memory (block 609 in Figure 5A). This is explained in detail regarding Figure 13. Finally, at stage 1090 the stored data are optionally provided for visualization, for example at GUI 611 in a computer monitor 700. This is explained in detail regarding Figure 14. The execution of the method for time step t finishes at stage 2000.

Figure 7 shows a flow diagram representing how data is collected from respective sources, as introduced in stages 1010a, 1010b and 1010c in Figure 6. In particular, Figure 7 refers to collecting data by data fetching and gathering means 601a (Figure 5A) from the production scheduling/planning information source 602a. These tasks are implemented trough stages 1010a to 1019a. Although not shown for conciseness, similar flow diagrams apply, mutatis mutandis, to data fetching and gathering means 601b and 601c, aiming at getting data from laboratory analysis information source 602b and real-time, production control operational information source 602c, respectively. In other words, Figure 7 shows how data is collected by any one of blocks 601a, 601b and 601c. It is next described with reference to block 601a. Execution starts at time step t (stage 1010a), where a secure connection to information source 602a is attempted (stage 1011a). At stage 1012a, it is checked whether the secure connection is established or not: if not, then the execution of all subsequent stages and associated tasks for current time step t is ignored and the execution of the method for time step t+1 is started; if yes, then the execution of tasks continues. At stage 1013a, it is checked whether (t=0) or (t>0): if (t=0), then a request for fetching all historical data is done (1014a); if (t>0), then a request for fetching the last data record is done (1016a). At stage 1015a/1017a, it is checked whether any data are gathered or not: if not, then the execution of all subsequent stages and associated tasks for current time step t is ignored and the execution of the method for time step t+1 is started; if yes, then the execution of tasks continues. At stage 1018a, the data gathered at stage 1014a/1016a are data frame-formatted and stored in memory means 609 in the computer system 600, such as in a principal memory or RAM (Random Access Memory), such that subsequent method stages can consume them. At stage 1019a collecting data finishes for time step t. The same method applies, mutatis mutandis, to data collection from information sources 602b and 602c, accomplished at stages 1010b and 1010c, respectively (see Figure 6). By selecting the preferred variables (model features), an accurate and robust predictive model 607 is later obtained.

Figure 8 shows a flow diagram representing the data processing tasks, accomplished by first information processing means 603a (Figure 5A), aiming at removing outliers and erroneous data from, and, filling gaps and missing data in production scheduling/planning information source 602a. These tasks are implemented trough stages 1020a to 1027a. Although not shown for conciseness, similar flow diagrams apply, mutatis mutandis, to first information processing means 603b and 603c, aiming at removing outliers and erroneous data from, and, filling gaps and missing data in laboratory analysis information source 602b and real-time, production control operational information source 602c, respectively. In other words, Figure 8 shows the details of the data processing tasks accomplished at any one of blocks 603a, 603b and 603c. It is next described with reference to block 603a. Execution starts at time step t (stage 1020a). Firstly, at stage 1021a, the data frame stored in memory means 609 at previous stage 1010a, is read. Then, it is checked (stage 1022a) whether outliers or erroneous data exist or not, for which statistical analysis techniques are preferably used. If yes, then the identified outliers and/or erroneous data are removed (stage 1023a); if not, then the execution of tasks continues. Secondly, it is checked (stage 1024a) whether gaps or missing data exist or not, for which time series robustness analysis techniques are preferably used. If yes, then the identified gaps and/or missing data are filled (stage 1025a), for which data imputation techniques are preferably used; if not, then the execution of tasks continues. In other words, this independent (separate from the processing of the other two processing means) processing at respective blocks 603a, 603b, 603c includes removing eventual outliers and erroneous data from the corresponding collected data and, if required, filling gaps and missing data in the respective source (602a, 602b, 602c). At block 603c, the values of real-time production control operational variables, collected from source 602c, are preferably averaged. At stage 1026a, the resulting data (processed data) are kept in a data frame format and stored in memory means 609, such as in a main memory or RAM, so that subsequent stages can use this data. Data processing for time step t finishes at stage 1027a.

Identification of the operational status (running or stopped) of the vacuum distillation unit is explained in detail with reference to Figure 9 and is accomplished through stages 1030 to 1034. Execution starts at time step t (stage 1030). Firstly, at stage 1031, values of one or several operational variables of the vacuum distillation unit 300 are read from source 602c and stored in memory means 609 at previous data processing stage 1020c. The operational variables whose values are read is preferably at least one temperature parameter measured in the vacuum unit (more preferably a temperature related to the main distillation column of the vacuum unit, such as the flash zone temperature in the vacuum unit) in order to determine whether the vacuum distillation unit is in operation or not. Optionally, at least one pressure parameter in the vacuum unit (more preferably a load pressure in the vacuum unit, such as the flash zone column pressure in the vacuum unit) and/or at least one load (flow rate) parameter in the vacuum unit (such as the load to vacuum unit) may also be read in order to determine whether the vacuum distillation unit is in operation or not. Secondly (stage 1032), the values of the selected operational variables are checked and processed. At this stage, a variable defining the status (running or stopped) of the vacuum distillation unit is defined from the selected operational variables. Preferably, it is checked whether the values of the read variables (such as the flash zone temperature in the vacuum unit and optionally the flash zone column pressure in the vacuum unit and/or the load to vacuum unit) are within a respective threshold or not, and depending on the result, it is determined that the vacuum distillation unit is running (in operation) or it is stopped. Thirdly (stage 1033), the defined variable (about the status of the vacuum distillation unit) is correspondingly stored in memory means 609, such that subsequent method stages, such as stage 1040, can use it. Identification of the operational status of the vacuum distillation unit 300 of the oil refinery 100 is then finish for time step *t* (stage 1034).

Figure 10 shows a flow diagram representing how the data independently processed at previous stages 1020a, 1020b and 1020c (Figure 6) and blocks 603a, 603b and 603c (Figure 5A) is jointly processed at block 604 in Figure 5A, in order to construct a principal, merged input dataset 605 (also referred to as main dataset 605) in the form of a time series. Main dataset 605 comprises first, auxiliary, merged input dataset 605a (first subset 605a), second, auxiliary, merged input dataset 605b (second subset 605b), and third dataset 605c (third subset 605c). These tasks are implemented trough stages 1050 to 1055. Execution starts at time step *t* (stage 1050). A possible implementation of these datasets 605a, 605b, 605c has already been described.

Firstly (stage 1051), a response time or time shift between certain information considered as input data to the vacuum distillation unit 300 (for example, the flash zone temperature in the vacuum distillation unit 300, the pressure of the vacuum distillation unit 300 at the flash region, and the load to the vacuum distillation unit 300) and output data from the vacuum distillation unit 300 is calculated. The output data is represented by the real value (analytically obtained in the laboratory) of the production operation parameter to be predicted, that is, the penetration value 305 of the residue 303 of the vacuum distillation unit 300. This is done because the production units of the oil refinery 100 in general, and the vacuum distillation unit 300 in particular, have/has a given volume and therefore a given hydraulic retention time (HRT) relating output flows to input flows, which eventually means that time relation between the penetration value 305, among other outputs, and input data to the vacuum distillation unit 300, is not instantaneous and must be determined. Thus, this time relation (referred to as response time or time shift) must be determined. This is preferably done by running a deep time correlation analysis between the input data to the vacuum distillation unit 300 and output data from the vacuum distillation unit 300, preferably finally validated by HRT analysis.

Secondly (stage 1052), the data corresponding to the real-time, production control operational information 602c, used for predicting a penetration value at prediction determination means 608, is averaged according to a time window determined by the response time or time shift previously calculated and the sampling time (at a frequency of preferably between several hours and a few days) of the real value of the (analytically obtained in the laboratory) penetration value 305. This averaging is required because the previously determined response time or time shift practically represents the latest, effective time from which changes in control operational variables 602c start having a considerable influence on the penetration value 305, and therefore, the values taken by operational variables 602c within the previously determined time window should be preferably considered, these being represented by their average value corresponding to said time window.

Thirdly (stage 1053), a main dataset 605 in the form of a time series is constructed by joining the data independently processed by first information processing means 603a, 603b and 603c. This is done through the application of time synchronization techniques based on the sampling time of the real value of the (analytically obtained in the laboratory) penetration value 305 of the residue 303 of the vacuum distillation unit 300, and the sampling time of the data corresponding to the real-time, production control operational information 602c represented by the previously averaged corresponding data. Data corresponding to production scheduling/planning information source 602a and laboratory analysis information source 602b (different from the actual penetration value) do not need to be considered for the time synchronization process as said data are static regarding the penetration value 305 of the residue 303 of the vacuum distillation unit 300, and therefore already synchronized with respect to it.

Finally (stage 1054), first subset 605a, second subset 605b and third subset 605c are constructed, derived from the principal, merged input dataset 605. These subsets 605a, 605b, 605c are in the form of a time series, arranged in a data frame format. A possible way of constructing these subsets have already been explained. They are stored in memory means 609, such that subsequent method stages can consume them. More specifically, first subset 605a will be used by predictive model construction means 606 at stage 1060 and information storage means 609 at stage 1080; second subset 605b will be used by prediction determination means 608 at stage 1070 and information storage means 609 at stage 1080; and production control operational variables dataset 605c will be optionally used by information storage means 609 at stage 1080 and visualization construction means 610 and therefore displaying means 700 at stage 1090.

First subset 605a contains the type of information (values of variables) needed for constructing a supervised learning model, that is to say, a plurality of samples of real values of the variable to be predicted, also called target variable (in this case, the penetration value 305 of residue 303 of vacuum distillation unit 300), and an equivalent number of real values of corresponding input variables considered as influencing, also called model features, obtained from sources 602a-602c. The real values of the target variable (penetration value 305) are obtained by applying the well-known, so-called needle penetration method performed through laboratory analysis. Therefore, the samples of these real values used for constructing the predictive model are included in the information source 602b representing laboratory analysis information. As a matter of example, because first subset 605a comprises historical data obtained from sources 602a-602c, a plurality of historical values of each involved parameter is typically comprised in this subset 605a.

In turn, model features are a plurality of input variables coming from information sources 602a, 602b and 602c (Figure 5A). Model features coming from information source 602a represent qualitative information about the crude oils 101 inputted to the oil refinery 100 as raw material. Preferably, such model features are the names of the specific crudes and their percentages as defined in the actual production plan, as disclosed with reference to Figure 5A. Model features coming from information source 602b are crude oil characterization features that represent quantitative information about the crude oils 101 inputted to the oil refinery 100 as raw material, as disclosed with reference to Figure 5A. Model features coming from information source 602c represent measurements captured with a plurality of sensors, actuators and controllers used for controlling operational variables of the vacuum distillation unit 300, such as temperature, pressure and flow rate, as disclosed with reference to Figure 5A. By selecting suitable model features, an accurate and robust predictive model 607 is obtained. In sum, first subset 605a contains the variables required to train, test and validate a stacking regression algorithm.

Among model features coming from source 602b, in addition to historical values of penetration value, in the order to build a prediction model for the penetration value, at least the amount of vacuum residue asphaltenes (%w) is preferably used. In a more preferred embodiment, at least one additional crude oil characterization feature is used, selected from the following ones: amount of crude oil sulphur (%w) and amount of atmospheric residue yield (%v). In a still more preferred embodiment, the crude oil characterization features used are the amount of vacuum residue asphaltenes (%w), the amount of crude oil sulphur (%w), the amount of atmospheric residue yield (%v) and the amount of heavy diesel yield (%v). Optionally, additional crude oil characterization features may be collected.

Among model features coming from source 602c, preferably, at least one temperature parameter and at least one flow rate (load) parameter are used. The at least one temperature parameter is preferably a temperature measured in the vacuum unit, more preferably a temperature related to the main distillation column of the vacuum unit. Still more preferably, this temperature is the flash zone temperature in the vacuum unit. The at least one flow rate (load) parameter is preferably the load to vacuum unit. It is remarked that any other temperature parameter or flow rate (load) parameter which is respectively correlated with the flash zone temperature in the vacuum unit or with the load to vacuum unit, may be respectively used instead. More preferably, at least one temperature parameter, at least one pressure parameter and at least one flow rate (load) parameter are used. The at least one temperature parameter is preferably a temperature measured in the vacuum unit, more preferably a temperature related to the main distillation column of the vacuum unit. Still more preferably, this temperature is the flash zone temperature in the vacuum unit (or a temperature parameter correlated thereto). The at least one flow rate (load) parameter is preferably the load to vacuum unit (or a flow rate (load) parameter correlated thereto). The at least one pressure parameter is preferably a load pressure in the vacuum unit, more preferably the flash zone column pressure in the vacuum unit (or a pressure parameter correlated thereto). Other load, pressure and temperature parameters may be additionally used for building a prediction model for the penetration value.

Data comprised in the second subset 605b corresponds to the same variables used to form the first dataset, except the variable representing the real values of the penetration 305 of the residue 303 of the vacuum unit 300. The record size of dataset 605b corresponds to the size of the last record of the input data to the vacuum distillation unit 300, for time step t. In other words, subset 605b contains the most updated values of the variables contained in subset 605a, except the penetration value of the residue, which is not included in subset 605b. It must be noted that, although the variables used to form the second dataset 605b and those used to form the first dataset 605a are the same, except the variable representing the real values of the penetration 305 of the residue 303 of the vacuum unit 300, which is not included in dataset 605b, the data contained in these two datasets are not the same: the data used to form the second dataset 605b corresponds to the last data record of the main dataset 605 at time t, while the data used to form the first dataset 605a corresponds to the historical data of dataset 605 up to the penultimate data record of the main dataset 605 at time t, that is, the up to last data record of the main dataset 605 at time (t-1).

Data in the third subset 605c comprises production control operational variables. They are preferably used for the later visualization in, for example, a graphical user interface 611. This visualization may contribute to validate the obtained predictions.

The record size of the principal dataset 605 depends on the record size of the datasets constructed by first information processing means 603a, 603b, 603c, that is, of the data frames correspondingly stored in memory means at stages 1020a, 1020b and 1020c. Therefore, at time step $t$, the record size varies depending on whether ($t$=0) or ($t$>0), as follows. If ($t$=0), then the record size of the main dataset 605 corresponds to the record size of all historical data available. In other words, the first time ($t$=0) the method is executed, the whole historical dataset is read. If ($t$>0), then the record size of the main dataset 605 preferably corresponds to the record size of the last data record. In other words, the whole historical dataset is available, but only the last sample is read, in order to gain efficiency. In turn, the record size of the first subset 605a and the production control operational variables dataset 605c depend on the record size of the main dataset 605 and therefore on time step t. Finally, the record size of the second subset 605b, used for example by prediction determination means 608 at stage 1070, for any time step t, is always the same: 1 record. The prediction of the penetration value 305 at time step t depends on the predictive model 607 at time step $t$ and on the last record of dataset 605 at time step *t,* that is, on the values of subset 605b on time step *t.* Therefore, the record size of the second subset 605b always corresponds to the record size of the last data record of dataset 605. Then, the jointly processing finishes for time step *t* (stage 1055).

Figure 11 shows a flow diagram representing the construction/upgrading of a predictive model 607 and corresponding model evaluation metrics calculation tasks, carried out by means 606, according to an embodiment of the invention. Such construction/upgrade is implemented through stages 1060 to 1068. Predictive model 607 is created by means of a computerized, numerical technique, based on machine learning algorithms, and applied to first subset 605a derived from the main dataset 605. Subset 605a represents a partial set of the main dataset 605, that is to say, subset 605a corresponds to a partial time period of the entire time period covered by the main dataset 605. In subset 605a, at least the most recent - in time- data sample is not included. In association with the constructed predictive model 607, some model evaluation metrics are calculated. Execution starts at time step *t* (stage 1060).

First (stage 1061), first subset 605a is read. The information (samples of real values of penetration value 305 and samples of real values of model features representing quantitative information about the crude oils 101 inputted to oil refinery 100 as raw material) coming from source 602b representing laboratory analysis information, used for constructing the predictive model, belongs to (or is obtained from) conventional crude oil laboratory assays regularly performed in the plant. Similarly, samples of real values of model features coming from information source 602a and samples of real values of model features coming from information source 602c, are also part of the production scheduling/planning information and real-time production control operational information, respectively, regularly managed in the plant. In other words, all the information used for constructing the predictive model of the invention is information created/obtained as a result of the regular, daily management of the plant. In other words, the information on which the method of the invention is based, is not specifically created for implementing the method.

Once the first subset 605a is read (stage 1061), the predictive model 607 is constructed (stage 1062). Depending on the time step *t,* the predictive model 607 is constructed for the first time (if *t*=0) or upgraded (if *t*>0). The predictive model 607 is constructed as follows: an optimum time window width $W_t$ is selected; model evaluation metrics are calculated considering the optimum time window width $W_t$; and an objective function is calculated. This process is represented as an optimization problem in which the free variable is the time window width $W_t$. The optimization algorithm is preferably a genetic algorithm. The optimum time window width $W_t$ has associated certain values of model evaluation metrics $MAE_{opt}$, $MSE_{opt}$ and $R^2_{opt}$, which are considered optimal, and where *MAE* stands for Mean Absolute Error, *MSE* stands for Mean Squared Error and $R^2$ stands for Coefficient of Determination. Thus, when (*t*=0), the following tasks are accomplished for construing the predictive model (stage 1063):

(i) for a model construction dataset *D*, where model construction dataset *D* is a subset of all historical data available of the first subset 605a, the optimum record size or time window width $W_{opt}$ of *D* is determined, where record size or time window width $W_t$ refers to the last $W_t$ data samples read from dataset 605a, at time step *t*. Preferably, for determining $W_{opt}$, a mathematical optimization problem is solved, which objective function is to minimize a weighted, multivariate function *F,* which is a function of *MAE, MSE* and $R^{-2}$, and in which the free variable is $W_t$; that is, $F = f$ (*MAE, MSE, $R^{-2}$).* In order to optimize $W_t$ (and therefore obtain $W_{opt}$), different model versions are created for different sizes of $W_t$, until the optimum $W_{opt}$ is obtained. Then, the model will always be built considering this time window $W_{opt}$, until this time window $W_{opt}$ is not optimal anymore and it needs re-optimization. In other words, the size of model construction dataset D is $W_{opt}$. While new real values of penetration are being read (and therefore incorporated into 605a), the content of dataset D is updated, but its size remains $W_{opt}$. This means that D is comprised of the last data sample plus $W_{opt}$-1 samples. The window is therefore a moving window.

(ii) calculating model evaluation metrics, such as standard, machine learning, regression model evaluation metrics *MAE* (Mean Absolute Error), *MSE* (Mean Squared Error) and $R^{-2}$ (Coefficient of Determination), in order to create the predictive model 607. The optimum goodness of fit metrics $G_{opt}$ of predictive model 607 is thus determined. Preferably, the mathematical algorithm used for solving the optimization problem (for obtaining an optimal value of $W_t$) is a genetic algorithm. The values of *MAE, MSE* and $R^{-2}$ for $W_{opt}$ are called $MAE_{opt}$, $MSE_{opt}$ and $R^{-2}_{opt}$, respectively. In other words, the values of *MAE, MSE* and $R^{-2}$ associated to the optimum value of $W_{opt}$ are preferably used. More specifically, a weighted key performance indicator $G_{opt}$, which is a function of $MAE_{opt}$, $MSE_{opt}$ and $R^{-2}_{opt}$, is determined, this representing the optimum goodness of fit metrics $G_{opt}$ of predictive model 607; that is, $G_{opt} = f$ (ature $MAE_{opt}$, $MSE_{opt}$, $R^{-2}_{opt}$). Once $W_{opt}$ and $G_{opt}$ are determined (stage 1063), the predictive model 607 for the penetration value 305 of the residue 303 has been constructed and can be used by subsequent stages.

The proposed prediction model applies regression algorithms based on machine learning techniques, in order to predict the vacuum penetration. In a preferred embodiment, the proposed prediction model is based on a regression model where a dataset *D* used for constructing the predictive model is divided into three additional partial datasets: a model training dataset *D'*; a model testing dataset *D''*; and a model validation dataset *D'''*. More specifically, the regression model is constructed based on an ensemble-learning meta-regressor for stacking regression method. Preferably, the prediction model is based on three different regression algorithms. Yet more specifically, the stacking regression method is based on three different and complementary regression model construction methods, respectively capable of catching: (i) the rapidly varying dynamics information contained within the model features coming from information source 602c; (ii) the quasi-static performance variation patterns information contained within the model features coming from information source 602b, and; (iii) the slowly varying information contained within the model features coming from information source 602a. The three different and complementary regression model construction methods may be respectively implemented by means of: (i) an adaptive filter algorithm; (ii) a neural network based regression algorithm, and; (iii) a gradient boosting based regression algorithm. The adaptive filter algorithm may be for example a *Recursive Least Squares* (RLS) algorithm, which is a very dynamic adaptive algorithm, capable of adapting to different perturbations that the system may suffer. It uses a few production variables to build the corresponding predictive model. The gradient boosting based regression algorithm (XGBOOST) is a regressor which produces several relatively weak predictive models in an iterative way (regression trees), which contribute to building a single robust predictor. It uses more production variables than the adaptive filter algorithm. The neural network (NN) represents a network comprising a set of functions, called neurons, organized in layers and connected between each other, wherein each neuron has computing capacity. Like XGBOOST, it uses more production variables than the adaptive filter algorithm.

It is remarked that with the information provided by the optimum goodness of fit metrics $G_{opt}$, operators of the refinery plant can reduce, in a controlled manner, the number of laboratory measurements for penetration value 305 regularly performed, and see the effect on the goodness of fit metrics $G_t$ of the corresponding predictive model 607 at time step $t$.

Turning back to Figure 11, when ($t$>0) and therefore predictive model 607 can be upgraded, every time a new, real penetration value 305 of residue 303 is obtained (stage 1064) from information source 602b of laboratory analysis information, and therefore this value is comprised in data subset 605a, the predictive model 607 and corresponding goodness of fit metrics $G_t$ are updated, for $W_t = W_{opt}$; while ($G_t \leq \rho G_{opt}$), then predictive model 607 and associated optimum record size or time window width $W_{opt}$ are considered optimal. Therefore, the number of laboratory measurements for penetration value 305 regularly performed can be reduced. In a preferred embodiment, $1 \leq \rho \leq 1.20$, such as $1 \leq \rho \leq 1.15$, or $1.02 \leq \rho \leq 1.15$, or $1.02 \leq \rho \leq 1.10$. For example, $\rho = 1.05$. If ($G_t > \rho G_{opt}$), then, a warning information may be created and displayed at the GUI 611, indicating that neither the predictive model 607 and associated optimum record size or time window width $W_{opt}$, nor the reduction accomplished for the number of laboratory measurements for penetration value 305 regularly performed, are considered optimal. When this happens, the tasks for determining $W_{opt}$ and $G_{opt}$ are newly executed. Eventually, once $W_{opt}$ and $G_{opt}$ are determined at stage 1063, it can be said that predictive model 607 for penetration value 305 of residue 303 has been constructed and can be used by subsequent stages.

When (t>0), at stage 1065, it is assumed that $W_{opt}$ and $G_{opt}$ are determined and therefore, an already existing predictive model 607 and corresponding goodness of fit metrics $G_t$ will be updated when a new, real penetration value 305 is obtained from information source 602b (stage 1064), where $G_t = f (MAE_t, MSE_t, R^{-2}_t)$. In this case, model construction dataset D will include the last data record read at time step $t$, that is, the content of dataset 605a at time step $t$, plus the ($W_{opt}$-1) previous data records read from dataset 605a, which at ($t$>0) can be fetched from information storage means 609. If ($G_t \leq \rho G_{opt}$), then the predictive model 607 and associated optimum record size or time window width $W_{opt}$ are considered optimal and no further action is taken (stage 1066, option YES). If ($G_t > \rho G_{opt}$), then, neither the predictive model 607 nor associated optimum record size or time window width $W_{opt}$ are considered optimal, so the tasks for determining $W_{opt}$ and $G_{opt}$ at stage 1063 are newly executed (stage 1066, option NO). Eventually, once $W_{opt}$ and $G_{opt}$ are newly determined at stage 1063, the predictive model 607 has been upgraded and can be used by subsequent stages.

Finally, predictive model 607 and corresponding model evaluation metrics, these including $W_{opt}$, $G_{opt}$ and $G_t$, correspondingly created/upgraded and calculated at stages 1063 to 1065, are all stored in memory means 609, such as RAM (stage 1067), so that subsequent stages can consume them. The construction or upgrading of the predictive model and calculation of model evaluation metrics is finished for time step $t$ (stage 1068).

Typically, a large historical database including data from sources 602a, 602b and 602c already exists at an oil refinery 100 at time step ($t$=0). This means that a large enough model construction dataset $D$ aiming at obtaining an optimum record size or time window width $W_{opt}$ at time step ($t$=0) will be available. In this case, the contribution of the adaptive filter algorithm, the gradient boosting based regression algorithm and the neural network based regression algorithm, respectively, to the stacking regression method, is optimally distributed. Nevertheless, the disclosed stacking regression method based on the above three different and complementary regression model construction algorithms, is also applicable to oil refineries 100 where no historical data or only a short historical data exists; in this case, the higher is the dynamics of the corresponding regression model construction algorithm, then the higher is its contribution to the stacking regression method, as the shorter is the optimum record size or time window width $W_{opt}$ needed by the corresponding algorithm for constructing a predictive model. More specifically, when no historical data or only a short historical data exists at oil refinery 100, then the construction of predictive model 607 is fully based or mostly based on the contribution of the adaptive filter algorithm, since the latter is the one of the three above regression model construction algorithms needing the shortest optimum record size or time window width $W_{opt}$ for constructing a predictive model. Yet more specifically, in a particular embodiment in which no historical data exits at oil refinery 100, a stacking regression method fully based on the adaptive filter algorithm starting off with an optimum time window width $W_{opt}$ of 1 (1 historical data record, the first existing historical data record of oil refinery 100) at time step ($t$=0) may be used for constructing predictive model 607. Then, as the oil refinery operates and therefore historical data are collected, the predictive model 607 is preferably constructed taking into account the contribution of the three mentioned algorithms -the adaptive filter algorithm, the gradient boosting based regression algorithm and the neural network based regression algorithm.

In sum, the construction of a predictive model 607 may comprise: applying supervised learning model construction techniques for building a regression predictive model; applying adaptive filtering techniques for catching the rapidly varying dynamics information contained within model features coming from information source 602c; applying gradient boosting based regression techniques for catching the slowly varying performance variation patterns information contained within the model features coming from information source 602a; applying neural network based regression techniques for catching the quasi-static information contained within the model features coming from information source 602b; calculating standard, machine learning, regression model evaluation metrics *MAE* (Mean Absolute Error), *MSE* (Mean Squared Error) and $R^2$ (Coefficient of Determination) for determining a goodness of fit metrics

$$G_t = f\ (MAE_t,\ MSE_t,\ R_t^2)$$ , for time step $t$; applying mathematical optimization techniques based on genetic algorithms for determining the optimum record size or time window width $W_{opt}$ of model construction dataset D. When no historical data exists at the oil refinery, the predictive model may be built using a stacking regression method fully based on adaptive filtering techniques starting off with an optimum time window width $W_{opt}$ of 1 at time step ($t$=0).

Therefore, by executing the algorithm represented in Figure 11, a prediction of the penetration value 305 of the residue of the vacuum distillation unit is obtained at a given time $t$, for any production operation mode (asphalt and non- asphalt production), for example covering the entire range of penetration values (up to tenths of a millimeter). In other words, the proposed method has large generalization capability, based on the regularly available production information of the plant, that its, production scheduling/planning information 602a, laboratory analysis information 602b and real-time production control operational information 602c. Besides, the proposed method allows the reduction of the number of regularly performed needle penetration method-based laboratory measurements of the penetration value, typically used for the regular operation of the plant.

Figure 12 shows a flow diagram including stages for performing a prediction of the penetration value of vacuum distillation residue, according to embodiments of the invention. This is performed at block 608 in Figure 5A. A prediction is obtained by applying the previously constructed predictive model 607 to the second subset 605b derived from the main dataset 605, which corresponds to a partial time period (preferably, the most recent one) of the entire time period covered by the main dataset 605. In subset 605b, at least the most recent -in time- data sample is included. Preferably, no data sample already included in the first subset 605a used for constructing the predictive model 607 is included in subset 605b.

Execution starts at time step $t$ (stage 1070). Firstly (stage 1071), the second subset 605b (current sample) and the predictive model 607 and model evaluation metrics $W_{opt}$, $G_{opt}$ and $G_t$, stored in memory means 609, are read. Secondly (stage 1072), the second subset 605b is inputted to the predictive model 607, such that the associated regression algorithm (such as a stacking regression algorithm) can determine a new predicted penetration value 305. Thirdly (stage 1073), the predictive model 607 is executed, and therefore the associated regression algorithm for the inputted data is also executed. As a consequence, a predicted penetration value 305 of residue 303 of the vacuum distillation unit 300 for time step $t$ is determined. Finally (stage 1074), the prediction is stored in memory means 609, such that subsequent method stages can consume it. The tasks of predicting a new penetration value for time step t are finished (stage 1075).

Figure 13 shows a flow diagram schematizing how to store in a persistent memory datasets 605a, 605b and 605c, the predictive model 607 and corresponding model evaluation metrics $W_{opt}$, $G_{opt}$ and $G_t$, and a prediction determined by the predictive model 607. Execution starts at time step $t$ (stage 1080). Firstly (stage 1081), all the information from memory means 609 (such as a RAM) is read. Secondly (stage 1082) the data is formatted such that it can be easily stored in persistent memory. This may be done as follows: (i) first, auxiliary, merged input dataset 605a and a timestamp value of corresponding time step $t$ are joined together in a first, single, merged system output dataset, yet more specifically in a first, single data frame format; (ii) second, auxiliary, merged input dataset 605b, the prediction determined by predictive model 607 and a timestamp value of corresponding time step $t$ are joined together, in a second, single, merged system output dataset, yet more specifically in a second, single data frame format; (iii) production control operational variables dataset 605c and a timestamp value of corresponding time step $t$ are joined together in a third, single, merged system output dataset, yet more specifically in a third, single data frame format; (iv) the evaluation metrics of the predictive model and a timestamp value of corresponding time step $t$ are joined together in a fourth, single, merged system output dataset, yet more specifically in a fourth, single data frame format. Finally (stage 1083), the formatted datasets and the predictive model 607 are correspondingly stored in persistent memory, such as in standard data storage format. If ($t$=0), then the corresponding data storage formats are created for the first time with the formatted datasets for time step $t$; if ($t$>0), then the corresponding data storage formats already exist and the formatted datasets for time step $t$ are appended to the previously existing formatted datasets. Regarding predictive model 607, preferably only its latest version (upgrade) is stored in persistent memory. The storing tasks for time step t finish at stage 1084.

In embodiments of the invention, the formatted datasets may be stored in standard, comma separated value format. The predictive model 607 may be stored in standard, model serialization pickle format. In embodiments of the invention, the formatted datasets and predictive model may be stored in standard, json format, or in standard, xml format, or in standard, database format. In embodiments of the invention, the persistent memory may be implemented as a secondary memory or hard drive; or as an external hard drive; or in the cloud.

In order to facilitate the supervision of the oil refinery plant by operators, prediction values - and other relevant data- continuously produced by the disclosed method and system, may be visually provided. Figure 14 summarizes how a prediction determined by predictive model 607, its model evaluation metrics and production control operational variables dataset 605c, can be displayed, for example at GUI 611, according to an embodiment of the invention. Dataset 605c is preferably displayed because it can be used as a reference to validate the predictive model (and the obtained predictions). Execution starts at time step t (stage 1090). Firstly (stage 1091), information is read from memory means, such as secondary memory or hard drive. Secondly (stage 1092), a GUI 611 is constructed and rendered by visualization construction means 610. This GUI represents the Graphical User Interface to be ultimately displayed by displaying means 700. GUI 611 includes the online, time evolution of the prediction value determined by the predictive model 607, predictive model evaluation metrics $G_{opt}$ and $G_t$, that is, the optimum goodness of fit metrics $G_{opt}$ of the predictive model and current predictive model goodness of fit metrics $G_t$, of time step $t$, and, the online, time evolution of the production control operational variables 605c, relevant to assess the penetration prediction. Prediction value 305 is preferably expressed in tenths of a millimeter. Together with the prediction value, a reference prediction value $P_{Ref}$, specified by the user, may be displayed. Preferably, additionally a first shadow of [$P_{Ref} \pm 2$] tenths of a millimeter representing the real, laboratory prediction value measurement repeatability error, plus, a second shadow of [$P_{Ref} \pm 3$] tenths of a millimeter representing the real, laboratory prediction value measurement reproducibility error, plus, the real laboratory measurements of the penetration value 305 made for the regular operation of the plant, are also shown in the corresponding time evolution plot. In addition to the line plot showing the time evolution of prediction value 305, its numerical value of time step $t$ is preferably also shown. Control operational variables 605c are preferably expressed in their corresponding measurement unit each. Predictive model evaluation metrics $G_{opt}$ and $G_t$, are preferably expressed in a form of a $G_t/G_{opt}$ ratio, where a ratio of ($G_t/G_{opt} \leq \rho$) represents an optimum goodness of fit metrics $G_t$ of predictive model 607, needing no further action to be taken, and, a ratio of ($G_t/G_{opt} > \rho$) represents a non-optimal goodness of fit metrics $G_t$ of predictive model 607, needing further action to be taken, which implies newly determining optimum record size or time window width $W_{opt}$ and optimum goodness of fit metrics $G_{opt}$ for predictive model 607 at stage 1060, that is, upgrading predictive model 607. Time may be expressed in a date-time format and a moving time window corresponding to the last ten days of production which can be moved backwards and forwards may also be shown. Finally (stage 1093), the constructed and rendered GUI 611 is sent to displaying means 700, such that the GUI can be displayed on it. Non-limiting examples of displaying means 700 are a computer monitor, a laptop computer screen, a tablet computer screen and a mobile phone screen. Displaying tasks for time step t finishes at stage 1094.

From the prediction penetration values obtained by the method and system of the invention, an action can be made on the refinery plant 100. For example, in the event the predicted value deviates from a target value more than a certain amount, a decision is made on the plant 100. Such decision may imply actuating on one or more actuating means in order to modify one or more operation variables. For example, operators may adjust one or more process control variables in a fast and dynamic manner, in order to obtain a desired penetration value immediately. Optionally, such actuation may be performed automatically, that is to say, without direct human intervention. For example, the control variables on which it may be actuated are a temperature parameter, a pressure parameter and/or a load parameter, such as the flash temperature of the column of the vacuum distillation unit 300, the pressure of the vacuum distillation unit 300 at the flash region and the load to vacuum distillation unit 300.

In sum, a method and system for online monitoring an oil refinery have been disclosed. They enable improving the performance of the vacuum distillation unit by achieving an optimum value of the penetration value of the residue of the vacuum distillation unit in a shortest time. Optionally, they enable actuating on a parameter of the oil refinery for example when the predicted penetration value departs from an expected value. Thanks to the soft sensor of the invention, in which the penetration value is monitored online, plant operators obtain a continuous measurement of the penetration value, instead of having to wait for a laboratory analysis measurement, which can take in average four to five hours. Therefore, operators are able to adjust process control variables in a fast and dynamic manner. In other words, a fast, dynamic and key decision-making tool has been provided, such that the desired penetration value is obtained in the shortest time. The proposed method and system also enables improving the performance of the coker unit of an oil refinery by achieving an optimum operation of the coker unit in a shortest time, because determining the optimum value of the penetration value in a shortest time allows plant operators to take the coker unit to its optimum operation point also in the shortest time. Therefore, the coker unit works in optimum conditions in longer periods of time, thus improving its performance. As a consequence, the performance of the oil refinery is also improved.

Furthermore, the method enables a reduction in the number of regularly performed needle penetration method-based laboratory measurements of the penetration value 305 of the residue 303 of the vacuum distillation unit 300, typically performed for supervising the regular operation of the plant. Such reduction is possible thanks to the reliable predictor (soft sensor) that continuously provides penetration predictions.

The method and system of the invention have been validated in production, in a real oil refinery plant, in which a system implemented according to system 500 has been integrated and validated in production and the penetration value of the residue has been monitored online according to the disclosed method every 15 minutes. An example of implementation in a real oil refinery plant is explained next.

A method and system according to the invention have been developed for monitoring a real oil refinery plant. A system for continuously monitoring the penetration value of the vacuum residue and a prediction model for predicting the penetration of vacuum residue have been built from: historic production planning/scheduling data (combination of crudes and respective percentages as defined in each production plan); a set of crude oil characterization features including the amount of crude oil sulphur (%w), the amount of heavy diesel yield (%v), the amount of atmospheric residue yield (%v) and the amount of vacuum residue asphaltenes (%w), in addition to historical (real) values of penetration value; and a set of production control operational variables including the flash zone temperature in the vacuum unit, the flash zone column pressure in the vacuum unit and the load to vacuum unit.

A predictive model has been built from this data, using a meta-regressor comprising an adaptive filter, a neural network and a gradient boosting-based regression algorithm. The following optimal values have been obtained: $W_{opt}$ = 250, $MAE_{opt}$ = 2.510, $MSE_{opt}$ = 10.508 and $R^2_{opt}$ = 0.515. A GUI has been built, that shows, at least: (i) the continuous 15-minute prediction of the penetration of the vacuum residue in a 10-day moving time window which is also updated with a 15-minute frequency, represented together with the actual values of the penetration obtained in the laboratory during the time period corresponding to the current moving window, a first shadow of $[P_{Ref} \pm 2]$ tenths of a millimetre representing the actual value of the repeatability error of the penetration value in the laboratory and a second shadow of $[P_{Ref} \pm 3]$ tenths of a millimetre representing the actual value of the reproducibility error of the penetration value in the laboratory, where $P_{Ref}$ = 12; (ii) the $G_{opt}$ and $G_t$ evaluation metrics of the predictive penetration model at each point in time; and (iii) the time evolution of at least the flash temperature of the vacuum unit, the pressure of the vacuum unit in the flash region and the load to the vacuum unit.

The predictive model has been applied to a data set composed of the same variables as the data set used to build the predictive model (excluding the actual value of the vacuum residue penetration), over a time period of 16 weeks, which is also updated with a frequency of 15 minutes, so that each time the data set is updated, a new prediction of the vacuum residue penetration is made and the corresponding verification actions for updating the window size ($W_{opt}$) are performed for updating the predictive model and the predictive model indicators $MAE_{opt}$, $MSE_{opt}$ and $R^2_{opt}$. During the application period, it has been verified that the updated values of $MAE_t$, $MSE_t$ and $R^2_t$ at each moment have always been close to the previously indicated optimum values of $MAE_{opt}$ = 2.510, $MSE_{opt}$ = 10.508 and $R^2_{opt}$ = 0.515, so that at all times the expression ($G_t \leq \rho G_{opt}$) has been maintained, for a value of $\rho$ = 1.05. During the application period of 16 weeks, refinery's process operation technicians have been able to verify the reliability of the monitoring system for predicting the penetration of the vacuum residue indicated above.

Once the monitoring method and system have been proved reliable, the predictions which are continuously -in this case, every 15 minutes- obtained, are used to optimize the production of the oil refinery plant. In other words, the predictions of the soft sensor start to be actively used to optimize the oil refinery plant. For example, the obtained predictions are continuously taken into account in order to decide whether or not a production control operational variable should be modified. For example, during execution of the following production planning:

| Crude unit | Mb/d crude | C1/C2 %v | Composition of load to crude %v | | | | | | | SUM |
|---|---|---|---|---|---|---|---|---|---|---|
| | | | AMENAM | RONCADOR HEAVY | BASRAH | PEREGRINO | MAYA | ESSIDER | SANKOFA | |
| C1 | 140 | 63.6 | 13.8 | 15.1 | 32 | 26.1 | 10.7 | 1.8 | 0.41 | 100 |
| C2 | 80 | 36.4 | 60.7 | 36.3 | | | 0.5 | 0.2 | 2.3 | 100 |
| C1+C2 | 220 | 100 | 31.5 | 23 | 20 | 16.3 | 6.9 | 1.2 | 1.1 | 100 |

in which the following crude characterization parameters were considered: the amount of crude oil sulphur (%w) was 2.15 %w, the amount of heavy diesel yield (%v) was 0.08 %v, the amount of atmospheric residue yield (%v) was 0.55 %v and the amount of vacuum residue asphaltenes (%w) was 15.87 %w,

the mentioned predictive model was used and 15-minute predictions were carried out, as well as corresponding verification actions for updating the window size $W_{opt}$ for updating the predictive model and the indicators $MAE_{opt}$, $MSE_{opt}$ and $R^2_{opt}$ of the predictive model. For example, the value of the vacuum residue penetration provided by the predictive model at an instant $t$ was 14 mm/10. The values of the flash temperature of the vacuum unit, the pressure of the vacuum unit in the flash region and the load to the vacuum unit were, at that instant t, 393.2 °C, 41.31 mmHg.a and 462.41 m³/h, respectively. The last known value of the laboratory measurements of the vacuum residue penetration, obtained in the laboratory several hours before, was 13 mm/10, while this production plan is programmed to optimize the unit to 15 mm/10. Based on the value of 14 mm/10 provided by the predictive model, the process operation technicians decided to lower the flash temperature of the vacuum column by 1.0 °C (from 393.2 °C to 392.2 °C), and thus modify the operating conditions of the column to adapt them to an optimal operation. A subsequent experimental sample of vacuum residue penetration from the laboratory registered a value of 15 mm/10, which means that the plant has been optimized based on the online monitoring method and system of the invention. Other actions, such as modifying other production control operational variables, have additionally been carried out when required, taking advantage of the continuously obtained predictions.

Throughout this document, the word "comprises" and variants thereof (such as "comprising", etc.) must not be interpreted as having an exclusive meaning, in other words, they do not exclude the possibility of what is being described incorporating other elements, steps, stages, etc.

At the same time, the invention is not limited to the specific embodiments described herein and also extends, for example, to variants that may be embodied by an average person skilled in the art (for example, with regard to the choice of information sources, process data, algorithms, formats, devices, components, configuration, etc.), within the scope of what is inferred from the claims.

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

**Claims**

1. A computer-implemented method for monitoring an oil refinery plant (100) comprising:

   from a first dataset (605a) of historical production information of the oil refinery plant (100), wherein the first dataset (605a) of historical production information comprises a plurality of samples of a penetration value (305) of a residue (303) of a vacuum distillation unit (300) obtained from laboratory analysis, and a plurality of samples of variables comprising production scheduling information (602a), laboratory analysis information (602b) and production control operational information (602c), wherein the laboratory analysis information (602b) includes in addition to the penetration value (305) of the residue (303) of the vacuum distillation unit (300), at least one crude characterization parameter, and wherein the production control operational information (602c) includes at least one temperature parameter and at least one flow rate parameter of the vacuum distillation unit (300): constructing (1060, 606) a predictive model (607) by applying a computerized, numerical algorithm based on machine learning techniques; and calculating model evaluation metrics;
   obtaining a prediction value (1070) for the penetration value (305) of the residue (303) of the vacuum distillation unit (300) by applying the constructed predictive model (607) to a second dataset (605b) comprising a most recent data sample of information of the oil refinery plant (100), the second dataset (605b) comprising samples of the same variables used to form the first dataset (605a), except for the variable representing the penetration (305) of the residue (303) of the vacuum unit (300);
   depending on the obtained prediction value (1070), actuating or not on at least one production control operational variable of the oil refinery plant (100).

2. - The method of claim 1, wherein the method further comprises processing the historical production information of the oil refinery plant (100) for constructing the predictive model as follows:

   independently processing the production scheduling information (602a), the laboratory analysis information (602b) and the production control operational information (602c);
   jointly processing (1050, 604) the independently processed production information, thus constructing a principal dataset (605) comprising a plurality of data samples.

3. The method of claim 2, wherein the stage of jointly processing (1050, 604) the independently processed production information, comprises:

calculating a response time between information being input data to the vacuum distillation unit (300) and output data therefrom, the input data comprising at least a flash zone temperature of the vacuum distillation unit (300), the output data comprising a penetration value (305) of the residue (303) of the vacuum distillation unit (300) obtained from laboratory analysis;

averaging the data comprised in the production control operational information (602c) to be used for obtaining a prediction value (1070), according to a time window determined by said response time and to a sampling time of the penetration value (305) of the residue (303) of the vacuum distillation unit (300) obtained from laboratory analysis; and

joining the independently processed production information including the averaged data corresponding to the production control operational information source (602c), thus constructing the principal dataset (605) comprising a plurality of data samples.

4. The method of any one of claims 1-3, wherein said production scheduling information (602a) represents qualitative information about the crude oils (101) inputted to the oil refinery (100) as raw material, said qualitative information being the names of the crude oils (101) and their percentages.

5. The method of any one of claims 1-4, wherein the at least one crude characterization parameter included in the laboratory analysis information (602b) is one of: amount of crude oil sulphur (%w), amount of atmospheric residue yield (%v or %w), amount of vacuum residue asphaltenes (%w) and amount of heavy diesel yield (%v or %w).

6. The method of any one of claims 1-5, wherein the at least one temperature parameter of the vacuum distillation unit (300) is a temperature related to the main distillation column of the vacuum distillation unit (300), and the at least one flow rate parameter of the vacuum distillation unit (300) is the load to the vacuum distillation unit (300).

7. The method of claim 6, wherein the temperature related to the main distillation column of the vacuum distillation unit (300) is the flash zone temperature of the vacuum distillation unit (300).

8. The method of any one of claims 1-7, wherein the production control operational information (602c) further includes at least one pressure parameter of the vacuum distillation unit (300), said at least one pressure parameter of the vacuum distillation unit (300) preferably being the flash zone column pressure in the vacuum distillation unit (300).

9. The method of any one of claims 1-8, wherein the numerical algorithm based on machine learning techniques comprises the following stacking regression models: a first regression model applying adaptive filtering techniques for catching rapidly varying dynamics information contained within the production control operational information (602c); a second regression model applying neural network based regression techniques for catching quasi-static information contained within the laboratory analysis information (602b); and a third regression model applying gradient boosting based regression techniques for catching slowly varying information contained the production scheduling information (602a).

10. A system for monitoring an oil refinery plant (100), comprising:

means (606) for, from a first dataset (605a) of historical production information of the oil refinery plant (100), wherein the first dataset (605a) of historical production information comprises a plurality of samples of a penetration value (305) of a residue (303) of a vacuum distillation unit (300) obtained from laboratory analysis, and a plurality of samples of variables comprising production scheduling information (602a), laboratory analysis information (602b) and production control operational information (602c), wherein the laboratory analysis information (602b) includes in addition to the penetration value (305) of the residue (303) of the vacuum distillation unit (300), at least one crude characterization parameter, and wherein the production control operational information (602c) includes at least one temperature parameter and at least one flow rate parameter of the vacuum distillation unit (300): constructing (1060, 606) a predictive model (607) by applying a computerized, numerical algorithm based on machine learning techniques; and calculating model evaluation metrics;

means (608) for obtaining a prediction value (1070) for the penetration value (305) of the residue (303) of the vacuum distillation unit (300) by applying the constructed predictive model (607) to a second dataset (605b) comprising a most recent data sample of information of the oil refinery plant (100), the second dataset (605b) comprising samples of the same variables used to form the first dataset (605a), except for the variable representing the penetration (305) of the residue (303) of the vacuum unit (300);
means for, depending on the obtained prediction value (1070), actuating or not on at least one production control operational variable of the oil refinery plant (100).

**11.** - The system of claim 10, further comprising:

means (601a, 601b, 601c) for collecting said historical information of the oil refinery plant (100);
means (603a) for processing the production scheduling information (602a);
means (603b) for processing the laboratory analysis information (602b);
means (603c) for processing the production control operational information (602c);
means (604) for jointly processing the independently processed production information, thus constructing a principal dataset (605) comprising a plurality of data samples.

**12.** The system of any one of claims 10-11, further comprising:

storage means (609) for storing production information of the oil refinery plant (100), the predictive model (607) and corresponding model evaluation metrics, and the obtained prediction value; and
means (700) for displaying at least the obtained prediction value, said means (700) being one of a computer monitor or a laptop computer screen, or a mobile phone screen, providing a graphical user interface (611).

**13.** The system of any one of claims 10-12, which is embodied by a single, common computer system (600), or embodied by several, connected computer systems (600).

**14.** A computer program product comprising computer program instructions which, when executed by a computer, cause the computer to carry out the method of claim 1.

**15.** A computer-readable medium comprising instructions which, when executed by a computer, cause the computer to carry out the method of claim 1.

**Patentansprüche**

**1.** Computer-implementiertes Verfahren zum Überwachen einer Ölraffinerieanlage (100), umfassend:

aus einem ersten Datensatz (605a) historischer Produktionsinformationen der Ölraffinerieanlage (100), wobei der erste Datensatz (605a) historischer Produktionsinformationen eine Vielzahl von Werten eines Penetrationswerts (305) eines Rückstands (303) einer Vakuumdestillationseinheit (300) umfasst, wobei die Vielzahl der Werte aus Laboranalysen resultiert,
und
aus einer Vielzahl von Werten von Variablen, die Produktionsplanungsinformationen (602a), Laboranalyseinformationen (602b) und Informationen über die Produktionssteuerungsparameter (602c) umfassen,

wobei die Laboranalyseinformationen (602b) zusätzlich zu dem Penetrationswert (305) des Rückstands (303) der Vakuumdestillationseinheit (300) mindestens einen Parameter zur Charakterisierung des Rohöls umfassen,
und
wobei die Informationen über die Produktionssteuerungsparameter (602c) mindestens einen Temperaturparameter und mindestens einen Durchflussratenparameter der Vakuumdestillationseinheit (300) umfassen:

Konstruieren (1060, 606) eines Vorhersagemodells (607) durch Anwenden eines computergestützten, numerischen Algorithmus, der auf maschinellen Lernverfahren basiert;
und
Berechnen von Modellbewertungsmessgrößen;
Erhalten eines Vorhersagewertes (1070) für den Penetrationswert (305) des Rückstandes (303) der Vakuumdestillationseinheit (300) durch Anwenden des konstruierten Vorhersagemodells (607) auf einen zweiten Datensatz (605b), der einen jüngsten Datensatz von Informationen der Ölraffinerieanlage (100) umfasst, wobei der zweite Datensatz (605b) Werte der gleichen Variablen umfasst, die zur Bildung des ersten Datensatzes (605a) verwendet wurden, mit Ausnahme der Variablen, die den Penetrationswert (305) des Rückstands (303) der Vakuumdestillationseinheit (300) darstellt;

Einwirken oder Nicht-Einwirken auf wenigstens einen Produktionssteuerungsparameter der Ölraffinerieanlage (100) in Abhängigkeit von dem erhaltenen Vorhersagewert (1070).

2. Verfahren nach Anspruch 1, wobei das Verfahren ferner das Verarbeiten der historischen Produktionsinformationen der Ölraffinerieanlage (100) zum Erstellen des Vorhersagemodells wie folgt umfasst;

unabhängiges Verarbeiten der Produktionsplanungsinformationen (602a), der Laboranalyseinformationen (602b) und der Informationen über die Produktionssteuerungsparameter (602c) ;

gemeinsames Verarbeiten (1050, 604) der unabhängig verarbeiteten Produktionsinformationen, wodurch ein Hauptdatensatz (605) konstruiert wird, der eine Vielzahl von Daten umfasst.

3. Verfahren nach Anspruch 2, wobei der Schritt des gemeinsamen Verarbeitens (1050, 604) der unabhängig verarbeiteten Produktionsinformationen umfasst:

Berechnen einer Reaktionszeit zwischen Informationen, die Eingangsdaten für die Vakuumdestillationseinheit (300) sind, und Ausgabedaten davon,

wobei die Eingangsdaten mindestens eine Flammzonentemperatur der Vakuumdestillationseinheit (300) umfassen,

wobei die Ausgabedaten einen Penetrationswert (305) des Rückstandes (303) der Vakuumdestillationseinheit (300) umfassen, der aus einer Laboranalyse erhalten wird;

Mittelwertbildung der Daten, die in den Informationen über die Produktionssteuerungsparameter (602c) enthalten sind,

wobei die Informationen über die Produktionssteuerungsparameter (602c) zum Erhalten eines Vorhersagewerts (1070) verwendet werden,

wobei die Mittelwertbildung in Bezug zu einem Zeitfenster durchgeführt wird, das abgeleitet wird aus der berechneten Reaktionszeit und aus einer Abtastzeit des Penetrationswertes (305) des Rückstands (303) der Vakuumdestillationseinheit (300), der aus der Laboranalyse erhalten wird; und

Zusammenfügen der unabhängig verarbeiteten Produktionsinformationen einschließlich der gemittelten Daten, die den Informationen über die Produktionssteuerungsparameter (602c) entsprechen, um daraus den Hauptdatensatz (605) zu bilden, der eine Vielzahl von Daten umfasst.

4. Verfahren nach einem der Ansprüche 1 bis 3, wobei die Produktionsplanungsinformationen (602a) qualitative Informationen über die Rohöle (101) darstellen, die der Ölraffinerie (100) als Rohmaterial zugeführt werden, wobei die qualitativen Informationen die Namen der Rohöle (101) und ihre Prozentsätze sind.

5. Verfahren nach einem der Ansprüche 1 bis 4, wobei der mindestens eine Parameter zur Charakterisierung des Rohöls, der in den Laboranalyseinformationen (602b) enthalten ist, einer der folgenden ist: Menge an Schwefel im Rohöl (%w), Menge an atmosphärischen Rückständen (%v oder %w), Menge an Asphaltenen als Vakuumrückstände (%w) und Menge an "Schwerem Diesel" (%v oder %w).

6. Verfahren nach einem der Ansprüche 1 bis 5, wobei der mindestens eine Temperaturparameter der Vakuumdestillationseinheit (300) eine Temperatur ist, die sich auf die Hauptdestillationskolonne der Vakuumdestillationseinheit (300) bezieht, und der mindestens eine Durchflussparameter der Vakuumdestillationseinheit (300) die Belastung der Vakuumdestillationseinheit (300) ist.

7. Verfahren nach Anspruch 6, wobei die auf die Hauptdestillationskolonne der Vakuumdestillationseinheit (300) bezogene Temperatur die Temperatur der Entspannungszone der Vakuumdestillationseinheit (300) ist.

8. Verfahren nach einem der Ansprüche 1 bis 7, wobei die Informationen über die Produktionssteuerungsparameter (602c) ferner mindestens einen Druckparameter der Vakuumdestillationseinheit (300) umfassen, wobei der mindestens eine Druckparameter der Vakuumdestillationseinheit (300) vorzugsweise der Druck in der Entspannungszone der Vakuumdestillationseinheit (300) ist.

9. Verfahren nach einem der Ansprüche 1-8, wobei der auf maschinellen Lerntechniken basierende numerische Algorithmus die folgenden Stapelregressionsmodelle umfasst:

ein erstes Regressionsmodell, das adaptive Filtertechniken anwendet, um schnell variierende dynamische Informationen zu erfassen, die in den Informationen über die Produktionssteuerungsparameter (602c) enthalten sind;

ein zweites Regressionsmodell, das auf neuronalen Netzen basierende Regressionstechniken anwendet, um quasi-statische Informationen zu erfassen, die in den Laboranalyseinformationen (602b) enthalten sind; und

ein drittes Regressionsmodell, das auf Gradientenverstärkung basierende Regressionstechniken anwendet, um langsam variierende Informationen zu erfassen, die in den Produktionsplanungsinformationen (602a) enthalten sind.

10. System zur Überwachung einer Ölraffinerieanlage (100), umfassend:

Mittel (606), um

aus einem ersten Datensatz (605a) historischer Produktionsinformationen der Ölraffinerieanlage (100), wobei der erste Datensatz (605a) historischer Produktionsinformationen eine Vielzahl von Werten eines Penetrationswerts (305) eines Rückstands (303) einer Vakuumdestillationseinheit (300) umfasst, wobei die Vielzahl der Werte aus Laboranalysen resultiert, und

aus einer Vielzahl von Werten von Variablen, die Produktionsplanungsinformationen (602a), Laboranalyseinformationen (602b) und Informationen über die Produktionssteuerungsparameter (602c) umfassen,

wobei die Laboranalyseinformationen (602b) zusätzlich zu dem Penetrationswert (305) des Rückstands (303) der Vakuumdestillationseinheit (300) mindestens einen Parameter zur Charakterisierung des Rohöls umfassen, und

wobei die Informationen über die Produktionssteuerungsparameter (602c)

mindestens einen Temperaturparameter und

mindestens einen Durchflussratenparameter der Vakuumdestillationseinheit (300) umfassen:

ein Vorhersagemodell (607) zu konstruieren (1060, 606) durch Anwenden eines computergestützten, numerischen Algorithmus, der auf maschinellen Lerntechniken basiert; und

Modellbewertungsmetriken zu berechnen;

Mittel (608) zum

Erhalten eines Vorhersagewertes (1070) für den Penetrationswert (305) des Rückstandes (303) der Vakuumdestillationseinheit (300) durch Anwenden des konstruierten Vorhersagemodells (607) auf einen zweiten Datensatz (605b), der einen jüngsten Datensatz von Informationen der Ölraffinerieanlage (100) umfasst,

wobei der zweite Datensatz (605b) Werte der gleichen Variablen umfasst, die zur Bildung des ersten Datensatzes (605a) verwendet wurden, mit Ausnahme der Variablen, die den Penetrationswert (305) des Rückstands (303) der Vakuumdestillationseinheit (300) darstellt;

Mittel, um in Abhängigkeit von dem erhaltenen Vorhersagewert (1070) auf wenigstens einen Produktionssteuerungsparameter der Ölraffinerieanlage (100) einzuwirken oder nicht einzuwirken.

11. System nach Anspruch 10, das ferner Folgendes umfasst:

Mittel (601a, 601b, 601c) zum Sammeln der historischen Informationen der Ölraffinerieanlage (100);

Mittel (603a) zum Verarbeiten der Produktionsplanungsinformationen (602a);

Mittel (603b) zur Verarbeitung der Laboranalyseinformationen (602b);

Mittel (603c) zur Verarbeitung der Informationen über die Produktionssteuerungsparameter (602c) ;

Mittel (604) zur gemeinsamen Verarbeitung der unabhängig verarbeiteten Produktionsinformationen, wodurch ein Hauptdatensatz (605) gebildet wird, der eine Vielzahl von Daten umfasst.

**12.** System nach einem der Ansprüche 10-11, das ferner Folgendes umfasst:

Speichermittel (609) zum Speichern von Produktionsinformationen der Ölraffinerieanlage (100), des Vorhersagemodells (607) und entsprechender Modellbewertungsmessgrößen sowie des erhaltenen Vorhersagewertes; und
Mittel (700) zum Anzeigen zumindest des erhaltenen Vorhersagewertes, wobei die Mittel (700) ein Computermonitor, ein Laptop-Computerbildschirm oder ein Mobiltelefonbildschirm sind, die eine grafische Benutzeroberfläche (611) aufweisen.

**13.** System nach einem der Ansprüche 10-12, das durch ein einziges, gemeinsames Computersystem (600) realisiert ist oder durch mehrere verbundene Computersysteme (600).

**14.** Computerprogrammprodukt, das Computerprogrammanweisungen enthält, die, wenn sie von einem Computer ausgeführt werden, den Computer veranlassen, das Verfahren nach Anspruch 1 auszuführen.

**15.** Computerlesbares Medium, das Anweisungen enthält, die, wenn sie von einem Computer ausgeführt werden, den Computer veranlassen, das Verfahren nach Anspruch 1 auszuführen.

## Revendications

**1.** Procédé informatisé de surveillance d'une raffinerie de pétrole (100) comprenant :

à partir d'un premier ensemble de données (605a) d'informations historiques de production de la raffinerie de pétrole (100), dans lequel le premier ensemble de données (605a) d'informations historiques de production comprend une pluralité d'échantillons d'une valeur de pénétration (305) d'un résidu (303) d'une unité de distillation sous vide (300) obtenue à partir d'une analyse en laboratoire, et une pluralité d'échantillons de variables comprenant des informations de programmation de la production (602a), des informations d'analyse en laboratoire (602b) et des informations opérationnelles de contrôle de la production (602c), les informations d'analyse en laboratoire (602b) comprenant, outre la valeur de pénétration (305) du résidu (303) de l'unité de distillation sous vide (300), au moins un paramètre de caractérisation du brut, et les informations opérationnelles de contrôle de la production (602c) comprenant au moins un paramètre de température et au moins un paramètre de débit de l'unité de distillation sous vide (300) : construire (1060, 606) un modèle prédictif (607) en appliquant un algorithme numérique informatisé basé sur des techniques d'apprentissage automatique ; et calculer des paramètres d'évaluation du modèle ;
obtenir une valeur prédictive (1070) pour la valeur de pénétration (305) du résidu (303) de l'unité de distillation sous vide (300) en appliquant le modèle prédictif construit (607) à un deuxième ensemble de données (605b) comprenant un échantillon de données le plus récent d'informations de la raffinerie de pétrole (100), le deuxième ensemble de données (605b) comprenant des échantillons des mêmes variables utilisées pour former le premier ensemble de données (605a), à l'exception de la variable représentant la pénétration (305) du résidu (303) de l'unité de vide (300) ;
en fonction de la valeur prédictive obtenue (1070), actionner ou non au moins une variable opérationnelle de contrôle de la production de la raffinerie de pétrole (100).

**2.** Procédé selon la revendication 1, dans lequel le procédé comprend en outre le traitement des informations historiques de production de la raffinerie de pétrole (100) pour construire le modèle prédictif de la façon suivante :

traiter indépendant les informations de programmation de la production (602a), les informations d'analyse en laboratoire (602b) et les informations opérationnelles de contrôle de la production (602c) ;
traiter conjoint (1050, 604) les informations de production traitées indépendamment, en construisant ainsi un ensemble de données principal (605) comprenant une pluralité d'échantillons de données.

**3.** Procédé selon la revendication 2, dans lequel l'étape de traitement conjoint (1050, 604) des informations de production traitées indépendamment comprend :

calculer un temps de réponse entre des informations qui sont des données d'entrée dans l'unité de distillation sous vide (300) et des données de sortie de celle-ci, les données d'entrée comprenant au moins une température de la zone de détente de l'unité de distillation sous vide (300), les données de sortie comprenant une valeur de pénétration (305) du résidu (303) de l'unité de distillation sous vide (300) obtenue à partir d'une analyse en laboratoire ;

calculer la moyenne des données comprises dans les informations opérationnelles de contrôle de la production (602c) à utiliser pour obtenir une valeur prédictive (1070), selon une fenêtre temporelle déterminée par ledit temps de réponse et un temps d'échantillonnage de la valeur de pénétration (305) du résidu (303) de l'unité de distillation sous vide (300) obtenue à partir d'une analyse en laboratoire ; et

joindre les informations de production traitées indépendamment, y compris les données moyennées correspondant à la source d'informations opérationnelles de contrôle de la production (602c), en construisant ainsi l'ensemble de données principal (605) comprenant une pluralité d'échantillons de données.

4.  Procédé selon l'une des revendications 1 à 3, dans lequel lesdites informations de programmation de la production (602a) représentent des informations qualitatives sur des pétroles bruts (101) introduits dans la raffinerie de pétrole (100) en tant que matière première, lesdites informations qualitatives étant les noms des pétroles bruts (101) et leurs pourcentages.

5.  Procédé selon l'une des revendications 1-4, dans lequel ledit au moins un paramètre de caractérisation du brut inclus dans les informations d'analyse en laboratoire (602b) est l'un parmi : une quantité de soufre du pétrole brut (%w), une quantité de rendement de résidus atmosphériques (%v ou %w), une quantité d'asphaltènes de résidus sous vide (%w) et une quantité de diesel lourd (%v ou %w).

6.  Procédé selon l'une des revendications 1-5, dans lequel ledit au moins un paramètre de température de l'unité de distillation sous vide (300) est une température liée à la colonne de distillation principale de l'unité de distillation sous vide (300), et ledit au moins un paramètre de débit de l'unité de distillation sous vide (300) est la charge de l'unité de distillation sous vide (300).

7.  Procédé selon la revendication 6, dans lequel la température liée à la colonne de distillation principale de l'unité de distillation sous vide (300) est la température de la zone de détente de l'unité de distillation sous vide (300).

8.  Procédé selon l'une des revendications 1 à 7, dans lequel les informations opérationnelles de contrôle de la production (602c) comprennent en outre au moins un paramètre de pression de l'unité de distillation sous vide (300), ledit au moins un paramètre de pression de l'unité de distillation sous vide (300) étant de préférence la pression de la colonne de la zone de de détente dans l'unité de distillation sous vide (300).

9.  Procédé selon l'une des revendications 1 à 8, dans lequel l'algorithme numérique basé sur des techniques d'apprentissage automatique comprend l'empilement suivant de modèles de régression : un premier modèle de régression appliquant des techniques de filtrage adaptatif pour capturer des informations dynamiques à variation rapide contenues dans les informations opérationnelles de contrôle de la production (602c) ; un deuxième modèle de régression appliquant des techniques de régression basées sur un réseau neuronal pour capturer des informations quasi-statiques contenues dans les informations d'analyse en laboratoire (602b) ; et un troisième modèle de régression appliquant des techniques de régression basées sur l'augmentation du gradient pour capturer des informations à variation lente contenues dans les informations de programmation de la production (602a).

10. Système de surveillance d'une raffinerie de pétrole (100), comprenant :

des moyens (606) pour, à partir d'un premier ensemble de données (605a) d'informations historiques de production de la raffinerie de pétrole (100), le premier ensemble de données (605a) d'informations historiques de production comprenant une pluralité d'échantillons d'une valeur de pénétration (305) d'un résidu (303) d'une unité de distillation sous vide (300) obtenue à partir d'une analyse en laboratoire, et une pluralité d'échantillons de variables comprenant des informations de programmation de la production (602a), des informations d'analyse en laboratoire (602b) et des informations opérationnelles de contrôle de la production (602c), les informations d'analyse en laboratoire (602b) comprenant, outre la valeur de pénétration (305) du résidu (303) de l'unité de distillation sous vide (300), au moins un paramètre de caractérisation du brut, et les informations opérationnelles de contrôle de la production (602c) comprenant au moins un paramètre de température et au moins un paramètre de débit de l'unité de distillation sous vide (300) : construire (1060, 606) un modèle prédictif (607) en appliquant un algorithme numérique informatisé basé sur des techniques d'apprentissage automatique ; et calculer les paramètres d'évaluation du modèle ;

des moyens (608) pour obtenir une valeur prédictive (1070) pour la valeur de pénétration (305) du résidu (303) de l'unité de distillation sous vide (300) en appliquant le modèle prédictif construit (607) à un deuxième ensemble de données (605b) comprenant un échantillon de données le plus récent des informations de la raffinerie de pétrole (100), le deuxième ensemble de données (605b) comprenant des échantillons des mêmes variables utilisées pour former le premier ensemble de données (605a), à l'exception de la variable représentant la pénétration (305) du résidu (303) de l'unité de vide (300) ;

des moyens pour, en fonction de la valeur prédictive obtenue (1070), actionner ou non au moins une variable opérationnelle de contrôle de la production de la raffinerie de pétrole (100).

11. Système selon la revendication 10, comprenant en outre :

des moyens (601a, 601b, 601c) pour collecter lesdites informations historiques de la raffinerie de pétrole (100) ;
des moyens (603a) pour traiter les informations de programmation de la production (602a) ;
des moyens (603b) pour traiter les informations d'analyse en laboratoire (602b) ;
des moyens (603c) pour traiter les informations opérationnelles de contrôle de la production (602c) ;
des moyens (604) pour traiter conjointement les informations de production traitées indépendamment, en construisant ainsi un ensemble de données principal (605) comprenant une pluralité d'échantillons de données.

12. Système selon l'une quelconque des revendications 10 à 11, comprenant en outre :

des moyens de stockage (609) pour stocker des informations de production de la raffinerie de pétrole (100), le modèle prédictif (607) et les paramètres d'évaluation de modèle correspondants, ainsi que la valeur prédictive obtenue ; et
des moyens (700) pour afficher au moins la valeur prédictive obtenue, lesdits moyens (700) étant l'un parmi un écran d'ordinateur ou d'ordinateur portable, ou un écran de téléphone mobile, fournissant une interface utilisateur graphique (611).

13. Système selon l'une quelconque des revendications 10 à 12, qui est réalisé par un seul système informatique commun (600), ou par plusieurs systèmes informatiques connectés (600).

14. Produit de programme informatique comprenant des instructions de programme informatique qui, lorsqu'elles sont exécutées par un ordinateur, permettent à ce dernier de mettre en oeuvre le procédé de la revendication 1.

15. Support lisible par ordinateur comprenant des instructions qui, lorsqu'elles sont exécutées par un ordinateur, permettent à l'ordinateur de mettre en oeuvre le procédé de la revendication 1.

**FIG. 1**

**FIG. 2**

**FIG. 3**

**FIG. 4**

**FIG. 5A**

**FIG. 5B**

**FIG. 5C**

FIG. 5D

FIG. 5E

**FIG. 5F**

**FIG. 5G**

Start 1000

1010a  1010b  1010c

1020a  1020b  1020c

1030

1040

NO

YES

1050

1060

1070

1080

FIG. 6

1090

End 2000

```
                    ╭─────────╮
                    │  Start  │──── 1010a
                    ╰─────────╯
                         │
                         ▼
                  ┌─────────────┐
                  │    1011a     │
                  └─────────────┘
                         │
                         ▼
         NO         ╱─────────╲
    ◄──────────────◄   1012a   ◄
                    ╲─────────╱
                         │
                         ▼ YES
   ┌────────┐    (t=0) ╱───────╲ (t>0)   ┌────────┐
   │ 1014a  │◄─────────  1013a  ─────────►│ 1016a  │
   └────────┘         ╲───────╱           └────────┘
       │                                       │
       ▼                                       ▼
  NO ╱───────╲                           ╱───────╲ NO
 ◄───  1015a  ─                         ─  1017a  ───►
     ╲───────╱                           ╲───────╱
         │ YES                               │ YES
         │         ┌──────────┐              │
         └────────►│  1018a   │◄─────────────┘
                   └──────────┘
                        │
                        ▼
                   ╭─────────╮
              ────►│   End   │◄────
                   ╰─────────╯
                        │ ╲
                          ╲──── 1019a
```

# FIG. 7

FIG. 8

**1030**

Start

1031

1032

1033

End

**1034**

## FIG. 9

**1050**

Start

1051

1052

1053

1054

End

**1055**

## FIG. 10

**FIG. 11**

**1070**

Start

**1071**

**1072**

**1073**

**1074**

End

**1075**

**FIG. 12**

**1080**

Start

**1081**

**1082**

**1083**

End

**1084**

**FIG. 13**

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6477516 B1
- WO 2008135411 A1

- US 2014180650 A1

**Non-patent literature cited in the description**

- **JAIMES et al.** Prediction of the Penetration Grade and Softening Point of Vacuum Residues and Asphalts by Nuclear Magnetic Resonance and Chemometric Methods. *Energy Fuels,* 2019, vol. 33, 6264-6272

- **FARANDA et al.** Neural software to estimate vacuum residue quality. *PTQ,* 2002